# EUROPÄISCHE PATENTANMELDUNG

(11) **EP 4 265 735 A1**
(43) Veröffentlichungstag der Anmeldung: **25.10.2023**
(21) Anmeldenummer: 22212538.7
(22) Anmeldetag: 09.12.2022
(51) Int. Cl.: C12Q 1/6851

(54) **PCR-MULTIPLEXING DURCH ZIELSEQUENZ-UNABHÄNGIGE-REPORTERMOLEKÜLE MIT UNTERSCHEID-BAREN SIGNALSTÄKEN**

(30) Priorität: 22.04.2022 EP 22169459
(71) Anmelder: Hahn-Schickard-Gesellschaft für angewandte Forschung e.V., 78052 Villingen-Schwenningen (DE)
(72) Erfinder: Lehnert, Michael, 78052 Villingen-Schwenningen (DE); Calabrese, Silvia, 78052 Villingen-Schwenningen (DE); Markl, Anja, 78052 Villingen-Schwenningen (DE); Schlenker, Franziska, 78052 Villingen-Schwenningen (DE); Kipf, Elena, 78052 Villingen-Schwenningen (DE)
(74) Vertreter: Hertin und Partner Rechts- und Patentanwälte PartG mbB

(57) **Zusammenfassung**

Die Erfindung betrifft ein Verfahren zum spezifischen Nachweis von mindestens zwei Nukleinsäure-Zielsequenzen durch mindestens zwei Zielsequenz-Unabhängige-Reportermoleküle im selben Detektionskanal, wobei mindestens zwei Mediatorsonden, umfassend mindestens eine Sonden- und eine Mediatorsequenz, und mindestens zwei Zielsequenz-Unabhängigen-Reportermolekülen eines ersten und zweiten Typs, jeweils umfassend mindestens eine Markierung mit einem jeweiligen Signalintensitätsmaximum im selben Detektionskanal, eingesetzt werden, und wobei die mindestens zwei Nukleinsäure-Zielsequenzen mittels einer Nukleinsäure-Nachweisreaktion, aufgrund einer für sie charakteristischen Signalintensität und/oder Emissionsspektrum detektiert werden. Die Erfindung betrifft außerdem ein Kit, für die Durchführung des Verfahrens.

## Beschreibung

### TECHNISCHER HINTERGRUND

Präzise und sensitive Nukleinsäure-Nachweismethoden, als Weiterentwicklung der real-time PCR, insbesondere die digitale PCR, haben in den vergangenen Jahren die Molekular-Diagnostik revolutioniert, da sie bei der Detektion von seltenen Gentargets der Standard-PCR überlegen ist (Garcia-Murillas et al. 2015; Milbury et al. 2014). Bei der digitalen PCR (dPCR) wird der Reaktionsmix und die darin enthaltenen Nukleinsäuren (z.B. ctDNA oder DNA) auf tausende von Reaktions-Räume aufgeteilt, wodurch die Zielsequenzen voneinander separiert werden und deren Amplifikation und Detektion, insbesondere von seltenen Zielsequenzen, erleichtert wird. Eine gängige Methode ist hierbei das mikrofluidische Vertropfen des Reaktionsmixes mit der nachzuweisenden Nukleinsäure in Öl oder die Aufteilung auf feste Mikrokavitäten, wodurch jede gebildete Einheit einen abgeschlossenen Reaktionsraum für eine Amplifikation und Detektion, beispielsweise während einer PCR, bildet. Bei Anwesenheit einer Nukleinsäure-Zielsequenz in einem Tropfen wird diese durch die PCR (oder andere Amplifikationsmethoden) amplifiziert. Die einzelnen Amplifikate werden zumeist durch sequenzspezifische fluorogene Nukleinsäure-Sonden detektiert, wobei entsprechende Fluoreszenzsignale generiert werden. Neben der absoluten Quantifizierung hat die dPCR zudem den Vorteil, dass der Einfluss von Inhibitoren verringert. Da die Quantifizierung bei der digitalen PCR als Endpunktanalyse erfolgt kann über die Fluoreszenz-Signalstärke ein "Signalcluster" (auch als Population bezeichnet) im Datenraum einer bestimmten DNA oder cDNA-Zielsequenz zugeordnet werden, was eine alternative Art der Datenklassifizierung ermöglicht (Quan et al. 2018; Whale et al. 2016).

Ein relevanter Punkt der dPCR ist das Multiplexen, da in Proben unterschiedliche Nukleinsäure-Zielsequenzen häufig in sehr geringen Konzentrationen vorkommen und es von hoher Relevanz ist, innerhalb einer Probe so viele Zielsequenzen wie möglich zu detektieren. Meist verbreitete Multiplex-Verfahren in der dPCR basieren darauf, dass DNA-Reportermoleküle je DNA-Zielsequenz ein Fluoreszenzsignal in einem Spektralbereich generieren, welches dem Detektionsbereich eines Detektionskanals entspricht. Je geräteseitigem Detektionskanal kann somit eine DNA-Zielsequenz nachgewiesen werden. Hierdurch ist der Multiplexgrad eines Gerätes zunächst aber auf die Anzahl an verfügbaren Detektionskanälen limitiert. Aktuell auf dem Markt verfügbare dPCR Geräte besitzen zwischen 2-6 unterschiedliche Fluoreszenzdetektionskanäle, wodurch geräteseitig maximal 6 Zielsequenzen analysiert werden können. Ein großes Problem hierbei ist, dass sich Wellenlängen bei einer hohen Anzahl an Detektionskanälen überschneiden können und durch den so entstehenden Crosstalk eine geringere Spezifität und Sensitivität erreicht wird.

Ein Verfahren zur Erhöhung des Multiplex Grades nutzt kombinatorische Ansätze, z.B. durch eine Kombination von Taqman-Sonden und DropOff-Probes als Zielsequenz spezifische Reportermoleküle. In diesem Fall ergibt sich durch die Kombinatorik der durch diese Sonden generierten Signale ebenfalls ein höherer Multiplexing-Grad ( (Madic et al. 2018; Stilla Technologies; Corné et al. 2021)). Da auch hier die einzelnen Mutationen z.T. nur indirekt nachgewiesen werden, sind diese Methoden ebenfalls sehr komplex in ihrer Entwicklung und sehr anspruchsvoll in der Auswertung und eine Zertifizierung oder Übertragung auf neue Target-Panels dementsprechend aufwendig.

Darüber hinaus gibt es weitere Verfahren zur Steigerung des Multiplexing-Grades in der digitalen PCR, welches die Generierung zusätzliche Populationen im Datenraum zum Ziel haben. Ein US-Patent der Firma Bio-Rad (US9921154B2) beschreibt eine multiplex digitale PCR-Analyse von mehr Zielsequenzen als optische Kanäle zur Verfügung stehen unter Berücksichtigung weiterer Proben-spezifischer Aspekte. Das Patent offenbart jedoch keine neuen Assay-Merkmale oderVerfahren zur Generierung solcher Ziel-Sequenz-Populationen. Diese Verfahren können nach aktuellem Wissenstand nur in Fällen angewendet werden, wenn es *per se* zu einer deutlichen Mehrfachbelegung von Tropfen mit verschiedenen DNA-Zielsequenzen kommt oder in Fällen, in welchen Signale durch zielsequenzspezifische Reportermoleküle wie Taqman-Sonden oder Sequenz-ungerichtete interkalierdende Farbstoffe generiert werden, welche an sich jedoch keine Neuerung gegenüber dem Stand der Technik darstellen. Es ist beschrieben, dass unterschiedliche Zielsequenz spezifische Sonden zu diesem Zweck z.B. unterschiedliche Fluorophore als Markierung nutzen können oder mehrfache Fluorophormarkierungen tragen können, um mehrere Populationen im selben Detektionskanal zu generieren. Da im Grundzustand die Signale der hier beschriebenen Sonden durch ein FRET-Quenching mit geringer Effizienz unterdrückt werden, ist die initiale Signalunterdrückung vergleichsweise gering, weshalb eine effektive Modellierung der Signalstärke und somit eine Trennung der Populationen im Datenraum nur begrenzt möglich sind. Da diese markierten Sonden zudem Zielsequenz-spezifisch sind, ist eine umfangreiche Optierung deren Fluoreszenzeigenschaften sehr ineffizient, da jeweils neue markierte Sonden synthetisiert werden müssen und dieses Vorgehen bei einer Übertragung auf weitere Sequenzen neu gestartet werden muss. Ebenso ist die Mehrfachmodifizierung solcher Zielsequenzspezifischer Sonden mit Fluorophoren sehr komplex und erfordert ebenso eine Modifizierung mit weiteren Quenchern, um das Signal ausreichend im Grundzustand zu unterdrücken um unspezifische Signalpopulationen im Datenraum zu vermeiden. Solche Mehrfachmodifikationen sind von der Synthese sehr komplex und erfordern zumeist eine aufwendige Optimierung, weshalb sie bei Zielsequenz spezifischen Reportermolekülen selten eingesetzt werden, da dieses Vorgehen sehr ineffizient ist.

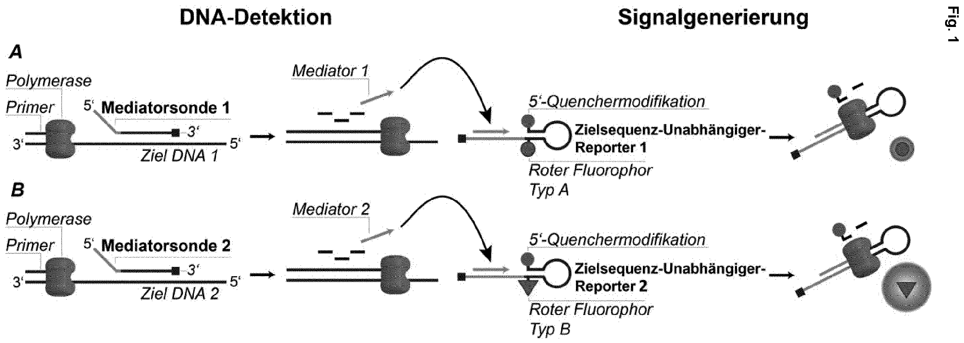

Die gängigste Variante bei diesem Intensity-Multiplexing ist daher die Variation der Konzentration der unterschiedlichen Typen von Zielsequenz spezifischen Reportermolekülen (z.B. Taqman Sonden) unter beibehalten einheitlicher und einfacher Fluoreszenzmarkierungen je Detektionskanal (Abb.1 rechts) (Pecoraro et al. 2016; Whale et al. 2016). Hierdurch können weitere Populationen im Datenraum eines Graphen entstehen. Aufgrund des Einsatzes von Zielsequenz spezifischen fluorogenen Reportermolekülen ist die Signalgenerierung direkt von der Zielsequenz bzw. dem PCR-System abhängig. Entsprechend besteht bei diesem direkten Nachweis das Problem eines hohen Optimierungsaufwandes, da es aufgrund des starken Rains (Datenpunkte welche zwischen verschiedenen Populationen liegen) zu unscharfen Signalpopulationen kommt, welche die Präzision verringern (Intensitäts-Multiplexing). Hierbei müssen die einzelnen PCR-Komponenten und insbesondere die fluorogenen Reporter mit identischen Fluoreszenzmarkierungen gut aufeinander abgestimmt werden, z.B. in orthogonalen Konzentrationsverhältnissen, was in der Praxis aber nicht immer möglich ist. Ergänzt werden kann dieses Verfahren auch durch Kombination mit weiteren Farbstoffen, welche einen weitere Fluoreszenzmarkierungen aufweisen, welche ihr Emissionsmaximum zwischen verschieden Fluoreszenzdetektionskanälen besitzen und hierdurch in mehreren Fluoreszenzkanälen gleichermaßen detektiert werden können. Hierdurch entstehen entsprechende Populationen zwischen den anderen Clustern im Datenraum, was mit zunehmender Dimension des Datenraums durch zusätzliche Detektionskanäle jedoch zunehmend abstrakt wird. Dies macht die Entwicklung entsprechender Multiplex-Nachweise und insbesondere deren Zulassung sehr aufwendig. Eine weitere Variation des Intensity-Multiplexings nutzt noch komplexere Konzentrationsvariationen um die Populationen im Datenraum zu versetzen und so weniger für Rain anfällig zu machen (Hughesman et al. 2016).

Eine Nachweismethode für Nukleinsäuren, welche im Gegensatz hierzu nicht Zielsequenz spezifische Reportermoleküle nutzt und auf einer Trennung von Nukleinsäure-Detektion durch Zielsequenz spezifischen Mediatorsonden und einer Signalgenerierung durch Zielsequenz unspezifischen Universal Reporter beruht, ist die Mediator Probe PCR, welche 2012 durch die Universität Freiburg patentiert wurde (WO2013079307A1, Faltin et al. 2012). Dieses Verfahren ist auf kolorimetrisches Multiplexing mit standardisierbaren Reportermolekülen ausgelegt. Hierbei wird je Detektionskanal ein Signal erfasst, welches unabhängig von seiner Signalstärke in einer homogenen Reaktion nur einem aktivierten Universal Reporter zugeordnet werden kann. Es wurde bereits beobachtet, dass unter real-time PCR Bedingungen unterschiedliche Fluorophore zu unterschiedlichen Signalintensitäten und Signalkurven führen können und durch eine Maximierung der Signalgenerierung der Zielsequenz unspezifischen fluorogenen universellen Reportermoleküle die Leistungscharakteristika der entsprechenden multiplex real-time PCRs optimiert werden können (Lehnert et al. 2018; Kipf et al. 2022). Eine Modellierung der Signalstärken in der real-time PCR hat aber keinen praktischen Mehrwehrt, da die Signalkurven im selben Detektionskanal durch diesen Ansatz nicht unterscheidbar sind. In der kolorimetrischen digitalen multiplex Mediator Probe PCR konnte zudem ergänzend gezeigt werden, dass eine Maximierung der Fluoreszenzsignalgenerierung gegenüber den Negativkontrollen zu einer besseren Auftrennung der Populationen im Datenraum führt (Schlenker et al. 2021b).

Im gleichen Zeitraum wurde eine Technologie der Seegene Inc. entwickelt, welche ebenfalls auf einer Trennung von DNA-Sequenz Detektion und Signalgenerierung über zwei Nachweismoleküle in der real-time PCR beruht. Hierbei bindet eine unmarkierte PTO-Sonde an eine DNA-Zielsequenz, wird gespalten und setzt ein Fragment frei, welches anschießend mit einem fluoreszenzmarkiertem Zielsequenz-unspezifischen Nachweismoleküle (CTO-Molekül), einem verlängerten Duplex bildet. Hierbei wird dieser Prozess dahingehend genutzt, um die Signalgenerierung über unterschiedliche Längen dieser Nachweismoleküle zu beeinflussen. Dies ermöglicht z.B. eine Differenzierung mehrerer Zielsequenzen im selben Detektionskanal, indem man bei definierten, vorab festgelegten Temperaturen das Signal ausliest. Ein weiteres Patent nutzt ebenfalls das Auslesen bei verschiedenen Temperaturen in der real-time PCR in einem Ansatz im selben Detektionskanal und kann entsprechend als Weiterentwicklung der oben genannten Technologie von Seegene interpretiert werden (PCT/CN2018/084794). Auch hierbei wird ein verlängertes Reportermolekül nach der Detektion aufgeschmolzen. Im Gegensatz zum vorherigen Patent kann hierbei ein Reportermolekül zur Differenzierung von verschiedenen Zielsequenzen genutzt werden. Da eine Temperierung bei der Auslese aktuell nicht in der dPCR in kommerziell erhältlichen Geräten standardmäßig umgesetzt werden kann, ist eine Übertragung auf die dPCR nicht direkt möglich.

Hahn-Schickard hatte sich bereits mit der Erhöhung des Multiplex-Grades in der PCR durch eine Generierung virtueller Fluoreszenzkanäle über das sogenannte Photobleaching befasst (WO2016087637A1) (Schuler et al. 2016). Um diesen Ansatz mit den Vorteilen einer Trennung von Signalgenerierung und Detektion der der digitalen Mediator Probe PCR zu kombinieren, wurden im Anschluss an eine digitale Mediator Probe PCR mit verschiedene Universal Reporter Typen mit unterschiedlichen Fluorophor-Markierungen ein Schritt hinzugefügt, in welchem diese durch Licht einer LED-Lampe gebleicht wurden (Bleaching) und im selben Fluoreszenzdetektionskanal ausgelesen. Je nach gewähltem Fluorophor ist durch das Bleaching eine Abnahme der Signalintensitäten zu beobachten, wodurch die Populationen im Datenraum eines Diagramms getrennt werden können und zusätzliche, sogenannte virtuelle Kanäle, generiert werden (Schlenker et al. 2021a). Da die Bleaching-Technologie jedoch zusätzliche Geräte und Prozessschritte benötigt, welche aktuell noch nicht mit kommerziell erhältlichen Geräten geliefert werden können, ist eine Umsetzung in bereits bestehenden dPCR Plattformen aufgrund technischer und regulatorischer Hürden nicht möglich. Die gewählten Fluorophore als Markierungen der Universal Reporter führten in diesem Ansatz jedoch vor dem Bleaching-Schritt noch nicht zu unterschiedlichen Fluoreszenz-signalstärken, welche eine einheitliche Trennung der Populationen im Datenraum eines Diagramms ermöglichten, wenn mehr als eine Zielsequenz je Reaktion enthalten war. Die eingesetzten Universal Reporter besaßen hierbei nicht die notwendigen Konfigurationen bei Fluorophoren und Quenchern, welche eine monochrome Identifizierung ermöglichen. Aufgrund der hohen Streuung der Fluoreszenzsignalintensitäten der Reportermarkierungen konnte so keine eindeutige Unterscheidung zwischen einer einzelnen Fluoreszenz-Signalpopulation mit höherem "Rain" und einer zweiten Signalpopulation im Datenraum ermöglicht werden. Insbesondere ermöglich diese Technologie keine augenscheinliche Auswertung, ob es sich in einer Multiplexreaktion um eine oder zwei Populationen handelt, und liefert somit keine Möglichkeit eines direkten Multiplexing im gleichen Detektionskanal durch Zielsequenz-unspezifische Reporter (Schlenker et al. 2021a).

### AUFGABE DER ERFINDUNG

Ansätze um die Signalstärken an Zielsequenz unabhängigen Reportermolekülen (generiert durch z.B. unterschiedliche Fluorophore und/oder Quencher) so einzustellen, dass in einer Reaktion mehrere unterscheidbare Signalcluster im selben Detektionskanal bzw. in entsprechenden Bereich des mehrdimensionalen Datenraums entstehen und hierdurch die Vorteile einer indirekten Signalgenerierung hinsichtlich der Assay-Präzision und Robustheit ausgenutzt werden um das zuvor beschriebene Problem des kolorimetrischen Multiplexings oder Zielsequenz spezifischen Intensity-Multiplexings in einer digitalen PCR zu umgehen, fehlen bisher im Stand der Technik. Bisherwurde lediglich beschrieben in einer digitalen Amplifikation durch Zielsequenz unspezifische Reportermoleküle monochrom zu multiplexen und hierdurch höhere Multiplexgerade zu erzielen, indem man zusätzliche Prozessschritte einführt (z.B. Bleaching). Dies verkompliziert den Ablauf bzw. die Auswertung, verringert die Präzision, führt zu einem zusätzlichen Optimierungsaufwand und erschwert beispielsweise Zulassungsverfahren. Daher ist es die Aufgabe der vorliegenden Erfindung die Anzahl an nachweisbaren Nukleinsäure-Zielsequenzen im Rahmen einer Nachweisreaktion, über die Anzahl an geräteseitigen Detektionskanälen hinaus, zu erhöhen.

### ZUSAMMENFASSUNG DER ERFINDUNG

Die vorliegende Erfindung löst diese Aufgabe durch das Verfahren der anhängigen und unabhängigen Ansprüche.

Daher bezieht sich die Erfindung in einem Aspekt auf ein Verfahren zum spezifischen Nachweis von mindestens zwei Nukleinsäure-Zielsequenzen durch mindestens zwei Zielsequenz-Unabhängige-Reportermoleküle im selben Detektionskanal, umfassend die Schritte:
a. Bereitstellen von mindestens einer ersten und einer zweiten Nukleinsäure-Zielsequenz,
b. Bereitstellen von mindestens einer ersten und einer zweiten Mediatorsonde, jeweils umfassend ein Oligonukleotid,
   wobei das Oligonukleotid einer ersten Mediatorsonde eine Mediatorsequenz, und eine Sonden-Sequenz umfasst, wobei die Mediatorsequenz eine Affinität zu einem Zielsequenz-Unabhängigen-Reportermolekül eines ersten Typs besitzt und die Sonden-Sequenz eine Affinität zu einer ersten Nukleinsäure-Zielsequenz aufweist,
   wobei das Oligonukleotid einer zweiten Mediatorsonde eine Mediatorsequenz, und eine Sonden-Sequenz umfasst, wobei die Mediatorsequenz eine Affinität zu einem Zielsequenz-Unabhängigen-Reportermolekül eines zweiten Typs besitzt und die Sonden-Sequenz eine Affinität zu einer zweiten Nukleinsäure-Zielsequenz aufweist,
   wobei die mindestens erste und zweite Mediatorsonden keine Markierungen, oder vorzugsweise keine signalerzeugenden Markierungen, besitzen,
c. Bereitstellen von mindestens zwei Zielsequenz-Unabhängigen-Reportermolekülen mindestens eines ersten und eines zweiten Typs jeweils umfassend mindestens eine Markierung mit einem jeweils messbaren Signal im selben Detektionskanal, und eine Nukleinsäuresequenz, welche jeweils eine spezifische Affinität zu mindestens einer Mediatorsequenz besitzt,
   wobei jeder Typ der mindesten zwei Zielsequenz-Unabhängigen-Reportermoleküle durch die jeweils mindestens eine Markierung dahingehend charakterisiert ist, dass diese jeweils eine Signalintensität besitzt, welche vorzugsweise für den jeweiligen Typ der Zielsequenz-Unabhängigen-Reportermoleküle einzigartig ist, und von den Signalintensitäten der Markierungen aller anderen Zielsequenz-Unabhängigen-Reportermolekül Typen unterscheidbar ist und eine direkte Zuordnung zu der jeweiligen Nukleinsäure-Zielsequenz ermöglicht,
d. Durchführen einer Nukleinsäure-Nachweisreaktion, wobei mindestens eine Mediatorsequenz mindestens einer ersten Mediatorsonde freigesetzt wird, wenn deren Sonden-Sequenz an mindestens eine erste Nukleinsäure-Zielsequenz bindet,
   wobei die mindestens eine freigesetzte Mediatorsequenz an das mindestens eine Zielsequenz-Unabhängige-Reportermolekül eines ersten Typs bindet, wobei durch die mindestens eine Markierung ein Signal erzeugt wird, welches durch seine Signalintensität und/oder Emissionsspektrum charakteristisch für die erste dem jeweiligen Zielsequenz-unabhängigen Reporter zugeordneten Nukleinsäure-Zielsequenz ist, an welche die mindestens eine Sonden-Sequenz der mindestens einen ersten Mediatorsonde gebunden hat,
   und optional wobei mindestens eine Mediatorsequenz mindestens einer zweiten Mediatorsonde freigesetzt wird, wenn deren Sonden-Sequenz an mindestens eine zweite Nukleinsäure-Zielsequenz bindet, wobei die mindestens eine freigesetzte Mediatorsequenz an das mindestens eine Zielsequenz-Unabhängige-Reportermolekül eines zweiten Typs bindet, wobei durch die mindestens eine Markierung ein Signal erzeugt wird, welches durch seine Signalintensität und/oder Emissionsspektrum charakteristisch für die zweite dem jeweiligen Zielsequenz-unabhängigen Reporter zugeordneten Nukleinsäure-Zielsequenz ist, an welche die mindestens eine Sonden-Sequenz der mindestens einen zweiten Mediatorsonde gebunden hat,
e. Detektion des/der unter Schritt d. erzeugten Signals/Signale, umfassend die Detektion des Signals/der Signale in einem Detektionskanal und/oder eine Analyse des/der Signale Signals, der Signalstärke(n) und/oder des Emissionsspektrums/-spektren des Signals/der Signale und/oder des/der so generierten Cluster(s) des Signals/der Signale in einem Datenraum, bevorzugt wobei sich die Signale unterschiedlicher Zielsequenz-unspezifischer Reportermoleküle ebenfalls über ihre Signalstärken in identischen Detektionskanälen oder den entsprechenden Bereichen eines Datenraums unterscheiden lassen.

In Ausführungsformen umfasst somit eine erste Mediatorsonde ein Oligonukleotid, welches selbst eine Mediatorsequenz, und eine Sonden-Sequenz umfasst. In Ausführungsformen umfasst somit ferner eine zweite Mediatorsonde ein Oligonukleotid, welches selbst eine Mediatorsequenz, und eine Sonden-Sequenz umfasst. Bevorzugt unterscheidet sich die Mediatorsequenz der ersten Mediatorsonde von der Mediatorsequenz der zweiten Mediatorsonde. Bevorzugt unterscheidet sich die Sonden-Sequenz der ersten Mediatorsonde von der Sonden-Sequenz der zweiten Mediator-sonde.

In anderen Worten, in Ausführungsformen des erfindungsgemäßen Verfahrens unterscheidet sich die Mediatorsequenz der ersten Mediatorsonde, von der Mediatorsequenz der zweiten Mediator-sonde, und die Sonden-Sequenz der ersten Mediatorsonde, von der Sonden-Sequenz der zweiten Mediatorsonde jeweils. Bevorzugt hat daher auch die Sonden-Sequenz der zweiten Mediatorsonde eine Affinität zu einer "zweiten" Nukleinsäure-Zielsequenz, welche sich von der "ersten" Nukleinsäure-Zielsequenz (z.B. in ihrer Nukleinsäuresequenz) unterscheidet, zu welcher wiederum die Sonden-Sequenz der ersten Mediatorsonde eine Affinität besitzt. Gleiches gilt auch für optional vorhandene und optional verwendete weitere (dritte, vierte, etc.) Mediatorsonden.

In Ausführungsformen umfasst Schritt c. das Bereitstellen von mindestens zwei Zielsequenz-Unabhängigen-Reportermolekülen mindestens eines ersten und eines zweiten Typs jeweils umfassend mindestens eine Markierung mit einem jeweils messbaren Signal im selben Detektionskanal und/oder einem jeweiligen Signalintensitätsmaximum im selben Detektionskanal, und eine Nukleinsäuresequenz, welche jeweils eine spezifische Affinität zu mindestens einer Mediatorsequenz besitzt.

Erfindungsgemäß unterscheiden sich eine "erste" Nukleinsäure-Zielsequenz und eine "zweite" Nukleinsäure-Zielsequenz bevorzugt in ihrer Nukleinsäuresequenz und/oder ihren epigenetischen Modifikationen. Gleiches gilt auch für optional vorhandene und optional zu detektierende weitere (dritte, vierte, etc.) Nukleinsäure-Zielsequenzen.

In bevorzugten Ausführungsformen des erfindungsgemäßen Verfahrens werden Zielsequenz-Unabhängige-Reporter in Form von markierten Oligonukleotiden Nukleinsäure-Zielsequenzen zugeordnet. Diese werden durch die Freisetzung eines weiteren Oligonukleotides im Rahmen der Nukleinsäure-Nachweisreaktion aktiviert und generieren hierdurch ein Signal. Unterschiedliche Reportermolekültypen sind jeweils mit unterschiedlichen Markierungen (z.B. Fluorophoren und/ oder Quenchern und/oder Fluorophore und Quencher in unterschiedlichen Anzahlen) modifiziert und besitzen unterschiedliche DNA-Sequenzen, welche die Zuordnung zu einer Zielsequenz indirekt ermöglichen. In identischen Detektionskanälen erzeugen in Ausführungsformen unterschiedliche Zielsequenz-Unabhängige-Reportermolekültypen aufgrund ihrer unterschiedlichen Markierungen Signale unterschiedlicher Signalstärken. Hierdurch können die Signale unterschiedlicher Reportermolekültypen mit jeweils unterschiedlichen Markierungen auch im selben Detektionskanal bzw. dem entsprechenden aufgespannten Datenraum (aufgespannt durch die Achsen in einer mehrdimensionalen Darstellung oder durch die unterschiedlichen Detektionskanäle in einer rein mathematischen Gegenüberstellung/Analyse und/ oder einer algorithmischen Analyse ) auf Basis der unterschiedlichen Signalintensitäten unterschieden und Zielsequenzen zugeordnet werden. Erfindungsgemäß können bevorzugt die Signale von mindestens zwei unterschiedlichen Reporterkomplexen im gleichen Detektionskanal oder dem entsprechenden Bereich eines Datenraums nachgewiesen werden. Hierbei ermöglicht die Tatsache, dass diese Reportermoleküle Zielsequenz unabhängig sind, erstmalig die Entwicklung von Reportermolekülen mit besonders gut unterscheidbaren Signalstärken innerhalb desselben Detektionskanals. Indem diese Zielsequenz-unabhängig sind können sie zudem geeignete Konfigurationen der Markierungen besitzen, welche eine solche Signalmodellierung effizient ermöglichen. Beispielsweise können deren Signale im initialen Zustand durch Kontakt-Quenching unterdrückt werden, wodurch sie im aktivierten Zustand höhere Unterschiede in der Signalgenerierung zeigen oder mehrfache Markierungen mit Fluorophoren und/oder Quenchern besitzen. Anwender können hierdurch die Anzahl in einer Reaktion nachweisbarer Zielsequenzen über die Anzahl an Detektionskanäle hinaus erhöhen, ohne zusätzliche Geräte kaufen bzw. Geräte erweitern zu müssen oder weitere aufwendige Prozessschritte zu implementieren, was einen enormen Mehrwert darstellt und eine hohe Kosteneffizienz gewährleistet. Bestehende Laborabläufe könnten beibehalten werden und bereits zertifizierte Geräte können weitergenutzt werden, ohne dass eine erneute Implementierung und ggf. Re-Zertifizierung notwendig wird. Weiterhin ermöglicht dies eine effiziente Optimierung dieser Zielsequenz-Unabhänigen-Reportermoleküle, da diese anschließend für weitere Zielsequenzen eingesetzt werden können und nicht von Neu, in Zielsequenzabhängigkeit, entwickelt und optimiert werden müssen. Somit erzielt die Erfindung eine erleichterte und effiziente Prozessoptimierung für die standartmäßige Detektion von verschiedenen Zielsequenzen und eine Reduktion des benötigten Arbeits- und Kostenaufwandes.

Das erfindungsgemäße Verfahren ermöglicht ein präzises effizientes (direktes) monochromes Multiplexing durch Zielsequenz-Unspezifische-Reportermoleküle. Es ermöglicht hierdurch das effiziente Zielsequenz unabhängige optimieren der Reportermoleküle und verbessert so die Auftrennung der Signalpopulationen im selben Detektionskanal oder dem entsprechenden Bereich des Datenraums. Hierdurch wird ein präziser Nachweis an zusätzlichen Nukleinsäure-Zielsequenzen, über die Anzahl an Detektionskanälen des Gerätes hinaus, ermöglicht. Dies wird ermöglicht, da durch den Einsatz von Zielsequenz unabhängigen Reportermolekülen eine symmetrische Mehrfachmarkierung mit Fluorophoren und Quenchern und/oder eine Markierung mit unterschiedlichen Fluorophormarkierung für denselben Detektionskanal in einer geeigneten Konfiguration und/oder eine Modellierung der Fluoreszenzsignalgenerierung, durch z.B. Kontakt-Quenching, gegenüber Zielsequenz spezifischen Reportermolekülen wesentlich vereinfacht und verbessert wird.

Dies geschieht unter Verwendung von Zielsequenz-Unabhängigen-Reportermolekülen (z.B. Universal Reporter), wobei es sich um markierte Oligonukleotide handelt, welche jeweils (indirekt) einer nachzuweisenden Nukleinsäure-Zielsequenz zugeordnet werden können und welche durch die Bindung eines freigesetzten weiteren Oligonukleotides im Rahmen einer Nukleinsäurenachweisreaktion (z.B. PCR oder digitaler PCR) aktiviert werden. Zur Unterscheidung der Signale, welche die Anwesenheit unterschiedlicher Nukleinsäure-Zielsequenzen repräsentieren/anzeigen, im selben Detektionskanal bzw. im entsprechenden Bereich des Datenraums (der graphischen Darstellung der detektierten Signale) werden mindestens zwei nachzuweisende Zielsequenzen je zwei unterschiedlichen Typen von Zielsequenz-Unspezifischen-Reportermolekülen zugeordnet, welche im selben Detektionskanal ein Signal mit unterschiedlicher Signalstärke generieren.

Ein Vorteil des erfindungsgemäßen Verfahrens ist die Möglichkeit mehrere Ziel-Nukleinsäuresequenzen in demselben Detektionskanal eines (PCR-)Gerätes, z.B. dem Kanal für die Detektion eines roten Fluoreszenzsignals, gleichzeitig zu detektieren. Diese gleichzeitige Detektion in einem einzigen Detektionskanal wird durch die Verwendung von unterschiedlichen Zielsequenz-Unspezifischen-Reportermolekülen erreicht, die jeweils Markierungen umfassen welche unterschiedliche Signale mit ähnlichen Fluoreszenz-Emissionsspektrum generieren und welche gleichzeitig im gleichen Fluoreszenz-Detektionskanal eines Gerätes, z.B. ein digitales PCR-Gerät, detektiert werden können und trotzdem unterscheidbar voneinander sind. Durch dieses Merkmal ermöglicht die Erfindung ein monochromes Multiplexing, nämlich die Detektion mehrerer Targets innerhalb eines einzigen ("monochrom") Fluoreszenzdetektionskanals (mit einem bestimmten Wellenlängenbereich).

In manchen digitalen PCR Verfahren des Standes der Technik werden in Emulsionstropfen eine erste oder eine zweite Zielsequenz, jeweils vereinzelt in einem Tropfen, nachgewiesen, indem zwei markierte Sonden eingesetzt werden, welche jeweils ein homogenes Signal generieren, das von unspezifischen Amplifikationen unterscheidbar ist. Im erfindungsgemäßen Verfahren werden keine markieren Sonden eingesetzt, was den Vorteil bringt, dass die markierten Reportermoleküle stärker optimiert werden können, als dies bei den bekannten Verfahren das Standes der Technik möglich wäre, da die erfindungsgemäßen Reportermoleküle anschließend für weitere Assays genutzt werden können. Zudem können die erfindungsgemäßen Reportermoleküle bevorzugt nur eine Konfiguration einnehmen in welcher es zum Kontakt-Quenching kommt. Durch diese bevorzugte Eigenschaft wird das (direkte) monochrome Multiplexing in der erfindungsgemäßen Form bevorzugt ermöglicht oder unterstützt.

Ein weiterer Vorteil des erfindungsgemäßen Verfahrens ist, dass sich die gewünschten Detektions-Eigenschaften durch die Wahl der jeweiligen verwendeten Reportermoleküle präzise einstellen lassen, welche unabhängig von der zu detektierenden Zielsequenz sind. Das einfach herzustellende und zu designende Verbindungsstück zwischen Reportermolekülen mit gewünschten Eigenschaften (z.B. Fluoreszenzstärke und/oder Farbe) sind die Mediator-Sonden, welche eine Sonden- und eine Mediatorsequenz umfassen. Für die Individualisierung der zu detektierenden Ziel-Nukleinsäure müssen lediglich die zwei unterschiedlichen Sequenzbestandteile der Mediatorsonde bereitgestellt werden. Diese können dann mit dem zur Mediatorsequenz passenden Zielsequenz-Unspezifischen-Reportermolekül kombiniert werden. Durch diese Unabhängigkeit kann ein gewünschtes spezifisches Kontakt-Quenching eingestellt werden, was die Detektion mehrerer unterscheidbarer Reportermolekül-Signale im gleichen Detektionskanal ermöglicht. Außerdem kann so die Optimierung der Signale unabhängig von zahlreichen Zielsequenzen einmalig durchgeführt werden. Zusätzlich ist das erfindungsgemäße Verfahren Ressourcen-schonend, da Reportermolekül-Sets für beliebig viele Nukleinsäure-Zielsequenzen wiederverwendet werden können, wodurch die initial einmalig aufwendige Entwicklung der kombinierbaren Reportermolekül-Signale sehr Arbeits- und Kosten effizient ist.

Außerdem ermöglicht das erfindungsgemäße Verfahren einen direkten Nachweis einer Nukleinsäure-Zielsequenz da dieses einer Daten-Population (Signalpopulation in der Auswertung/Analyse) direkt zugeordnet werden, und keine Kombinatorik erfordert.

Eine Möglichkeit unterschiedliche Signalstärken durch unterschiedliche Markierungen an Reportermolekülen zu erzeugen, ergibt sich z.B. aus den physikalischen Moleküleigenschaften der Markierungen, welche durch ein Kontakt-Quenching nach der Aktivierung des Zielsequenz-unspezifischen Reportermoleküls verstärkt werden:
In Ausführungsformen handelt es sich daher bei der mindestens einen Markierung eines Zielsequenz-Unabhängigen-Reportermoleküls um mindestens ein Fluorophor und/oder mindestens einen Quencher.

Eine weitere essentielle Eigenschaft dieser Zielsequenz unspezifischen Reportermoleküle bei der effizienten Generierung präziser unterscheidbarer Signalpopulationen ist es, dass eine Markierungs-Konfiguration gewählt wird, welche es ermöglicht Signale in einer Art und Weise zu erzeugen, dass ihre Signalstärken ausreichend robust, stabil und hierdurch präzise genug sind, sodass sie im Rahmen des Ausleseprozesses ohne weitere Prozessschritte (wie z.B. Bleichen) differenzierbar bleiben. Es zeigte sich, dass dies z.B. möglich ist, indem Konfigurationen entsprechend der Basisstruktur eines universellen Reporters oder entsprechende Abwandlungen einheitlich verwendet werden. Zielsequenz-Unspezifische-Reportermoleküle können in geeigneter Weise modelliert werden, wodurch unterschiedliche Signalstärken präzise im Detektionskanal einstellbar sind (Lehnert et al. 2018).

Folglich unterscheiden sich in Ausführungsformen unterschiedliche Zielsequenz-Unabhängige-Reportermolekül Typen durch die Signalintensität und/oder das Emissionsspektrum ihrer mindestens einen Markierung.

In Ausführungsformen umfasst die mindestens eine Markierung eines Zielsequenz-Unabhängigen-Reportermoleküls mindestens zwei Fluorophore und/oder zwei Quencher mit gleicher oder unterschiedlichem Emissionsspektrum und/oder gleicher oder unterschiedlicher Signalintensität.

In manchen Ausführungsformen unterscheidet sich ein Reportermolekül eines ersten Typs von dem eines mindestens zweiten Typs durch die Anzahl an Markierungen bzw. Anzahl an Fluorophoren und/oder Quenchern. Somit können unterschiedliche Typen von Zielsequenz-Unabhängigen-Reportermolekülen anhand der Signalstärke und/oder Intensität der bei ihrer Aktivierung freigesetzten Fluoreszenz unterschieden werden. Hierfür können unterschiedliche Typen von Zielsequenz-Unabhängigen-Reportermolekülen eine unterschiedliche Anzahl und/oder unterschiedliche Fluorophore (mit unterschiedlichen Emissionsspektren/Farben und/oder Intensitäten) und/oder Quencher oder jede Kombination davon umfassen.

In bevorzugten Ausführungsformen in denen die Markierung mindestens einen Fluorophor umfasst, wird dessen Fluoreszenzsignal bis zur Aktivierung des Reportermoleküls vorzugsweise durch Kontakt-Quenching, auch Kontakt-Löschung genannt, unterdrückt. Somit wird bevorzugt kein Signal durch ein nicht-aktiviertes Reportermolekül freigesetzt. Eine Aktivierung findet vorzugsweise durch die Bindung einer Mediatorsequenz am entsprechenden Zielsequenz-Unabhängigen-Reportermolekül und/oder durch eine anschließende Verlängerung einer bereits am Zielsequenz-Unabhängigen-Reportermolekül gebundenen Mediatorsequenz durch eine Polymerase während einer Nukleinsäure-Nachweisreaktion statt.

In Ausführungsformen umfasst daher die mindestens eine Markierung eines Zielsequenz-Unabhängigen-Reportermoleküls mindestens ein Fluorophor und mindestens einen Quencher, wobei zwischen dem mindestens einen Fluorophor und dem mindestens einen Quencher bevorzugt ein Kontakt-Quenching stattfindet, solange keine Mediatorsequenz am entsprechenden Zielsequenz-Unabhängigen-Reportermolekül bindet, oder solange eine gebundene Mediatorsequenz während der Nukleinsäure-Nachweisreaktion nicht verlängert wurde.

Dies ermöglicht z.B. im Rahmen einer digitalen DNA-Amplifikation und fluoreszenzbasierten Detektion den Nachweis zusätzlicher DNA-Zielsequenzen in einem dafür geeigneten Gerät, ohne das weitere technische Hilfsmittel notwendig werden oder die Konzentrationen von Reportermolekülen mit Fluoreszenzmarkierungen variiert werden müssen. Hierdurch können z.B. ähnliche Ergebnisse wie durch die digitale Mediator Probe PCR (MP-PCR) in Kombination mit dem Photobleaching generiert werden, ohne dass der Einsatz eines Bleichprozesses (z.B. über eine LED oder thermisch) notwendig wird, was den experimentellen Aufwand signifikant reduziert, vereinfacht und bereits in bestehenden Geräten umsetzbar macht. Somit stellt das erfindungsgemäße Verfahren entsprechend einen enorm hohen Mehrwert gegenüber der digitalen Mediator Probe PCR (MP-PCR) oder anderen Verfahren des Standes der Technik dar, da es direkt mit existierenden Plattformen kompatibel ist und eine mögliche diagnostische Zulassung eines spezifischen Nachweisverfahrens signifikant vereinfachen kann. Das erfindungsgemäße Verfahren bietet zusätzliche neue Freiheitsgrade in der Assay-Entwicklung, wobei die Syntheseprozesse für entsprechende Zielsequenz spezifische Sonden sich nicht zwangsweise verkomplizieren. Zudem kann so die Sensitivität eines erfindungsgemäßen Assays signifikant gesteigert werden, da je Reaktion zusätzliche Nukleinsäuresequenzen miterfassen kann. Ein weiterer Vorteil ist, dass ein geeignetes Set an Zielsequenz-Unspezifischen-Reportermolekülen Populationen stets in ähnlichen Bereichen des Datenraums generiert, wodurch Rückschlüsse auf den jeweils aktivierten Reporter unabhängig der Zielsequenzen innerhalb der Nachweisreaktion möglich sind und somit dasselbe Set mit unterschiedlichen Nukleinsäure-Zielsequenz-Panels ohne aufwendige Optimierung eingesetzt werden kann.

In Ausführungsformen des erfindungsgemäßen Verfahrens umfasst die Analyse des Signals, der Signalstärke und/oder des Emissionsspektrums des Signals unter Punkt e. die Darstellung und/oder Analyse des detektierten Signals abhängig von der Signalstärke und/oder dem Detektionskanal und/oder des Emissionsspektrums in einem durch die ausgewerteten Detektionskanäle aufgespannten Datenraum.

Im Kontext der vorliegenden Erfindung wird ein Datenraum vorzugsweise durch einen Plot, ein Diagramm oder einen Graphen der unterschiedlichen Detektionskanäle erzeugt oder durch eine rein mathematische und/oder Algorithmen- und/oder computerbasierte Auswertung, Vergleich oder Gegenüberstellung der unterschiedlichen Detektionskanäle, wobei die jeweiligen Fluoreszenzsignale der eingesetzten Zielsequenz-unspezifischen Reporter in diesem 1 - n dimensionalen Datenraum (je nach Anzahl der für die Auswertung benötigten Detektionskanäle) Datenpunkte generieren, welche sich zu Clustern (Gruppen, Ansammlungen) gruppieren können.

Bevorzugt ist im Kontext der vorliegenden Erfindung ein Datenraum der Raum, welcher in einem Plot, Diagramm oder Graph durch eine X-Achse und eine Y-Achse (zweidimensionaler Plot), und optional bei einem dreidimensionalen Graph zusätzlich durch eine Z-Achse, oder bei einem entsprechend höherdimensionalen Raum durch eine weitere Achse, aufgespannt wird und in dem die darzustellenden Daten (der detektierten Signale) dargestellt werden. In Ausführungsformen in denen auf den Achsen eines Graphs im Verfahren verwendete Detektionskanäle dargestellt werden, zum Beispiel die im roten Detektionskanals detektierten Signale auf derX-Achse und die im grünen Detektionskanals detektierten Signale auf der Y-Achse, kann der Datenraum auch als "von den Detektionskanälen aufgespannt" bezeichnet werden. Vorzugsweise ist es möglich die im Datenraum dargestellten detektierten Signale, welche vorzugsweise in Form von distinkten Signalpopulationen dargestellt werden können, einer Zielsequenz eindeutig zuzuordnen. Dieses ist vorzugsweise auch möglich, wenn mehrere, für jeweils unterschiedliche Zielsequenzen spezifische, Signale im gleichen Detektionskanal detektiert wurden.

In Ausführungsformen unterscheiden sich die gleichzeitig in einer PCR-Reaktion eingesetzten Reportermoleküle untereinander entweder durch unterschiedliche Arten (z.B. Farben, Emissionsspektra, und/oder Intensitäten) von Fluorophoren und/oder unterschiedlichen Arten von Quenchern und/oder durch die Anzahl der Markierungen auf den unterschiedlichen Reportermolekülen. In Ausführungsformen des erfindungsgemäßen monochromen Multiplexing werden mindestens zwei Ziel-Nukleinsäuren im gleichen Detektionskanal indirekt durch Reportermoleküle mit unterschiedlichen bzw. unterscheidbaren Signalen detektiert. Bei mehr als zwei zu detektierenden Ziel-Nukleinsäuren können in Ausführungsformen, die für den Nachweis der Ziel-Nukleinsäuren zu generierenden Signale, wenn nötig, auf mehrere Detektionskanäle aufgeteilt werden (und die Markierungen der unterschiedlichen Reportermolekül-Typen entsprechend ausgewählt werden), wobei bevorzugt mindestens zwei Ziel-Nukleinsäuren im gleichen Detektionskanal indirekt durch Reportermoleküle mit unterschiedlichen bzw. unterscheidbaren Signalen detektiert werden.

In Ausführungsformen können mindestens eine erste und eine zweite Nukleinsäure-Zielsequenz, oder auch mindestens eine erste, eine zweite und eine dritte, oder sogar eine vierte, fünfte, sechste, siebte, achte, neunte, zehnte, elfte, zwölfte, dreizehnte, vierzehnte, fünfzehnte, sechzehnte, siebzehnte, achtzehnte, neunzehnte, zwanzigste, einundzwanzigste oder sogar mehr Zielsequenzen nachgewiesen bzw. ihre Anwesenheit detektiert werden.

Entsprechend der Anzahl der zu detektierenden Nukleinsäure-Zielsequenzen werden mindestens eine erste, zweite, dritte, vierte, fünfte, sechste, siebte, achte, neunte, zehnte, elfte, zwölfte, dreizehnte, vierzehnte, fünfzehnte, sechzehnte, siebzehnte, achtzehnte, neun-zehnte, zwanzigste, einundzwanzigste oder sogar mehr Mediatorsonden, jeweils umfassend ein Oligonukleotid, dies jeweils umfassend eine Mediatorsequenz und eine Sonden-Sequenz, bereitgestellt.

Entsprechend werden je nach Anzahl der zu detektierenden Nukleinsäure-Zielsequenzen Zielsequenz-Unabhängigen-Reportermoleküle eines ersten, zweiten, dritten, vierten, fünften, sechsten, siebten, achten, neunten, zehnten, elfte, zwölfte, dreizehnte, vierzehnte, fünfzehnte, sechzehnte, siebzehnte, achtzehnte, neun-zehnte, zwanzigste, einundzwanzigste oder sogar mehr, Typs bereitgestellt und im erfindungsgemäßen Verfahren oder Kit wie hierin beschrieben verwendet.

Ein Fachmann versteht, wie er erfindungsgemäß Typen von Zielsequenz-Unabhängigen-Reportermolekülen und deren Markierungen kombinieren kann, um den Nachweis von gewünschten Zielsequenzen gemäß der Erfindung zu erreichen.

In Ausführungsformen umfasst die Markierung des mindestens einen Zielsequenz-Unabhängigen-Reportermoleküls mindestens zwei komplementäre gegenüberliegende Nukleobasen mit jeweils mindestens einer Markierung oder mindestens zwei um eine Basenposition gegenüber einer komplementären Basenpaarung versetzte gegenüberliegende Basen mit jeweils mindestens eine Markierung.

Die vorliegende Erfindung ermöglicht die gleichzeitige Detektion von mehreren Ziel-Nukleinsäuren (Multiplexing), wobei die Zahl der gleichzeitig detektierten Ziel-Nukleinsäuren größer sein kann als die Anzahl der im Detektionsgerät verfügbaren Detektionskanäle. Dieses wird dadurch ermöglicht, dass die jeweils verwendeten Reportermoleküle Markierungen umfassen, die trotz gleichzeitiger Detektion der Signale mehrerer unterschiedliche Reportermoleküle im gleichen Detektionskanal voneinander unterscheidbare Signale generieren und bei der Analyse als distinkte (nicht überlappende) Signalpopulationen im zwei-, oder dreidimensionalen Datenraum dargestellt werden können. In Ausführungsformen ermöglicht dies die Unterscheidung der Signale, welche die Präsenz unterschiedlicher Ziel-Nukleinsäuren anzeigen, trotz Detektion im gleichen Detektionskanal, sowie, in spezifischen Ausführungsformen, auch die Unterscheidung vom Hintergrundrauschen.

In Ausführungsformen des erfindungsgemäßen Verfahrens findet in Schritt d.-e. n unterschiedliche Nukleinsäure-Zielsequenzen durch n unterschiedliche Zielsequenz-Unabhängige-Reportermolekül Typen indirekt detektiert werden, wobei die Detektion des in Schritt d. erzeugten Signals in k Detektionskanälen statt,
wobei n > k ist und n ≥ 2 ist, und
wobei mindestens zwei unterschiedliche Zielsequenz-Unabhängige-Reportermolekül Typen in Schritt e. im gleichen Detektionskanal detektiert und/oder bei der Darstellung unter Punkt e. im gleichen Bereich eines Datenraums dargestellt werden.

Durch die Anwendung des erfindungsgemäßen Verfahrens ist es in Ausführungsformen möglich, im höherdimensionalen Datenraum (>3 Dimensionen, bei mehr als drei Detektionskanälen) eine Datenklassifizierung durchzuführen. Durch das erfindungsgemäße Verfahren kann die Anzahl an unterscheidbaren Zielsequenzen in einer digitalen PCR in Ausführungsformen mindestens gegenüber den zur Verfügung stehenden Detektionskanälen verdoppelt werden, sofern dies nicht perse durch geräteseitige technische Limitationen, wie einem möglichen Crosstalk (gegenseitige Beeinflussung von Fluoreszenzsignalen; fälschliche Assoziation eines Fluoreszenzsignals mit dem Signal eines anderen Fluorophors durch die fälschliche Detektion einer Fluoreszenz von einem benachbarten Detektionskanal) zwischen unterschiedlichen Detektionskanälen, eingeschränkt ist.

In Ausführungsformen wird ein Signal einer Markierung eines Reportermoleküls solange unterdrückt, gelöscht oder gequencht, bis eine Mediatorsequenz an das Reportermolekül bindet und vorzugsweise durch eine Polymerase im Rahmen einer Nukleinsäure-Amplifikationsreaktion verlängert wird. In Ausführungsformen wird durch die Polymerase eine räumliche Trennung, z.B. mindestens eines Fluorophors von mindestens einem Quencher, erreicht, sodass ein Signal generiert wird und detektiert werden kann.

In Ausführungsformen des erfindungsgemäßen Verfahrens wird das Signal der Markierung eines Zielsequenz-Unabhängigen-Reportermoleküls durch die Spaltung und/oder Auftrennung des Zielsequenz-Unabhängigen-Reportermolekül und/oder durch räumliche Trennung des mindestens einen Fluorophor und des mindestens einen Quencher erzeugt.

In Ausführungsformen ist das mindestens eine Zielsequenz-Unabhängige-Reportermolekül ein Oligonukleotid oder ein Oligonukleotid-Komplex ist.

In Ausführungsformen des erfindungsgemäßen Verfahrens umfasst die Nukleinsäure-Nachweisreaktion unter Schritt d. ein Amplifikationsverfahren für DNA und/oder cDNA.

In Ausführungsformen des erfindungsgemäßen Verfahrens ist die Nukleinsäure-Nachweisreaktion unter Schritt d. eine PCR, RT-PCR, RPA oder LAMP, und wobei im Rahmen der DNA-Amplifikation durch eine enzymatische Aktivität eines Biomoleküls eine Mediatorsequenz einer an eine Ziel-Nukleinsäure gebundene Mediatorsonde freigesetzt wird, welche darauf an ein Zielsequenz-Unabhängiges-Reportermolekül bindet, sodass ein Signal generiert wird.

In Ausführungsformen erfolgt die Detektion unter Schritt e. im Rahmen einer digitalen Amplifikation und/oder Signalgenerierung.

In Ausführungsformen des erfindungsgemäßen Verfahrens handelt es sich bei den Zielsequenz-Unabhängigen-Reportermolekülen um Universal Reporter und/oder modulare Reporterkomplexe und bei der (jeweils) mindestens einen freigesetzten Mediatorsequenz um eine Komponente einer Mediator Probe PCR oder Mediator Displacement LAMP.

In Ausführungsformen ist die Nukleinsäure-Zielsequenz aus einer Konvertierung eines anderen Biomoleküls in DNA-Sequenzinformationen entstanden.

In manchen Ausführungsformen kann die Anwesenheit und/oder Menge einer RNA Sequenz (eine Ausführungsform eines Biomoleküls) in einer Probe bestimmt werden, indem die RNA Sequenz durch eine reverse Transkriptions-Reaktion in cDNA umgeschrieben wird. Diese cDNA kann in Ausführungsformen im Rahmen des erfindungsgemäßen Verfahrens amplifiziert und durch ein Zielsequenz-Unabhängiges-Reportermolekül indirekt nachgewiesen werden.

In Ausführungsformen des erfindungsgemäßen Verfahrens umfasst die mindestens eine Markierung eine Markierung ausgewählt aus einer elektrochemisch aktiven und/oder einer magnetischen Markierung.

In Ausführungsformen des erfindungsgemäßen Verfahrens wird das Signal bei unterschiedlichen Temperaturen ausgelesen.

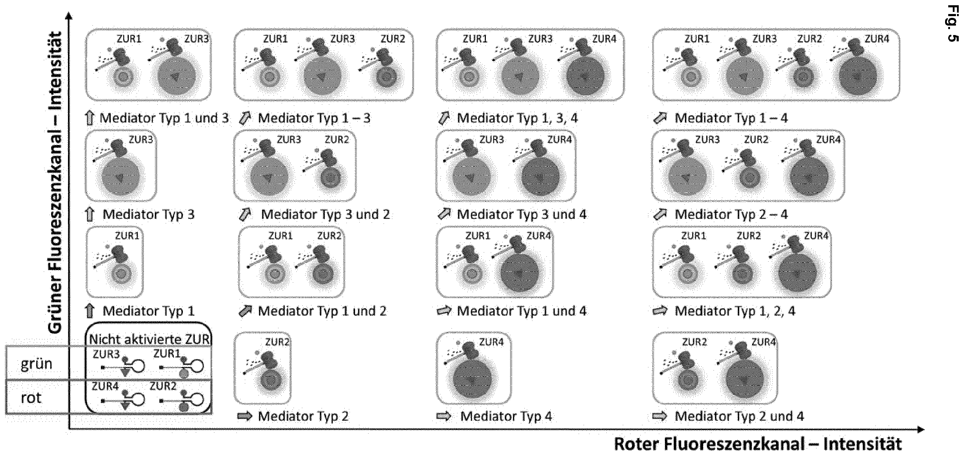

Bevorzugt ermöglicht das erfindungsgemäße Verfahren somit eine direkte Endpunktdetektion von n Targets in k-Detektionskanälen, wobei n > k und n ≥ 2. Im Gegensatz zum Stand der Technik werden Signale erfindungsgemäß nicht durch fluorogene Sonden, sondern durch Zielsequenz-Unabhängige-Reportermoleküle, welche auch als populations-spezifische Reporter beschrieben werden können, erzeugt, welche sich bevorzugt im Intensitätsbereich eindeutig unterscheiden. Dies gewährleistet eine größere Flexibilität im experimentellen Designprozess und ermöglicht die Verwendung von Reportermolekülen, welche Kontaktquenching zur anfänglichen Signalunterdrückung nutzen. Darüber hinaus ermöglicht es eine kontrollierte Signalerzeugung, was zu besser unterscheidbaren und robusteren Fluoreszenzsignalen führt, die sich besonders für die Endpunktdetektion eignen. Bevorzugte Ausführungsformen der vorliegenden Erfindung basieren auf universellen Reporterstrukturen und werden in Kombination mit Mediatorsonden für den Zielsequenznachweis verwendet. Eines der Grundprinzipien des erfindungs-gemäßen Verfahrens ist bevorzugt der Nachweis von n Ziel-Nukleinsäuresequenzen durch n zielspezifische Mediatorsonden-Typen, die n Reportermolekül-Typen aktivieren, wobei jeder Reportermolekül-Typ mit einem einzigartigen Fluorophor mit definierten optischen Eigenschaften markiert ist (Figur 1). In einem Beispiel einer Duplexreaktion würden zwei Fluorophore gewählt werden, die Fluoreszenz im gleichen Wellenlängenbereich emittieren, sich aber in ihrer Intensität im Detektionsbereich unterscheiden. Während der dPCR-Amplifikation werden die gebundenen markierungsfreien Mediatorsonden von der Polymerase gespalten und setzen eine Mediatorsequenz frei. Dieser Mediator aktiviert einen Reportermolekül-Typ, der dann ein Fluoreszenzsignal erzeugt. Die Summe aller Tröpfchen oder Kompartimente mit einem Signal eines bestimmten Reportermolekül-Typs bildet eine Population in einem bestimmten Bereich des Datenraums, entsprechend der eindeutigen Fluoreszenzmarkierung. Diejenigen Reportermolekül-Molekültypen, die mit Fluorophoren mit höherer Quantenausbeute markiert sind, erzeugen positive Populationen mit höheren Fluoreszenzintensitäten (Figur 1 unten: Hohe Intensitäten sind als größere Kreise dargestellt), und diejenigen mit niedrigeren Quantenausbeuten im Nachweisbereich erzeugen positive Populationen mit niedrigeren Fluoreszenzintensitäten (Figur 1 oben: Niedrige Intensitäten sind als kleinere Kreise dargestellt). Je nach ihrer Position im Datenraum können die gebildeten Populationen einer bestimmten Ziel-DNA-Sequenz zugeordnet werden, die in der Probe nachgewiesen werden soll. Bei Vorhandensein von zwei Targets in einem Kompartiment wird ein additiver Effekt der Fluoreszenzintensitäten angenommen, der zur Bildung einer dritten Population mit höherer Signalintensität führen würde. In weiteren Ausführungsformen könnte ein ähnlicher Signaleffekt z.B. durch die Verwendung von zwei verschiedenen Quencher-Typen oder zwei ähnlichen Fluorophor-Markierungen an einem Reportermolekül in einer Duplex-Reportermolekül-dPCR-Reaktion beobachtet werden. In einem Beispiel eines Vier-Plex-Reportermolekül-dPCR-Assays, der in zwei Detektionskanälen durchgeführt wird, führt das Vorhandensein mehrerer Targets in einem Kompartiment zur Bildung orthogonaler Populationen. Das bedeutet, dass durch die Verwendung von vier verschiedenen Reportermolekül-Typen theoretisch bis zu sechzehn verschiedene Populationen detektiert und differenziert werden könnten (z.B. Figur 5). Ohne an die Theorie gebunden zu sein, kann angenommen werden, dass bei digitalen Assays die Verteilung einer Zielnukleinsäure der Poisson-Statistik folgt. Somit könnte die Erzeugung von doppelt und mehrfach positiver Populationen von den Konzentrationen der Zielnukleinsäure, sowie von möglicherweise auftretenden konkurrierenden Effekten abhängen, die durch den Nachweis von Genloci verursacht werden, die sich die gleichen Primerpaare teilen. Im Vergleich zu Standes der Technik-Multiplexing-Methoden höherer Ordnung hat das vorliegende Verfahren den Vorteil, dass die Konzentrationen der Zielsequenz-Unabhängigen-Reportermoleküle in verschiedenen Assays konstant bleiben können. Dies gibt dem Assay-Design mehr Freiheit und die Fluoreszenzsignalpopulationen können mit hoher Effizienz optimiert werden. Bei der SNP-Detektion kommt ein weiterer Vorteil der Kombination von Mediatorsonden mit Zielsequenz-Unabhängigen-Reportermolekülen zum Tragen. Während des Elongationsprozesses werden bevorzugt gebundene Mediator-sonden hochspezifisch von der Polymerase gespalten, wobei der Mediatorteil von dem Sondenteil abgespalten wird. Die Spaltungsseite befindet sich bevorzugt zwischen dem ersten und zweiten Nukleotid des Sondenteils der Mediatorsonde. Nur wenn die Spaltung korrekt durchgeführt wurde, kann der freigesetzte Mediator vollständig an die Mediator-Bindungsseite am spezifischen Zielsequenz-Unabhängigen-Reportermolekül-Typ binden, um die Signalerzeugung einzuleiten. Wenn keine korrekte Spaltung stattgefunden hat, wird bevorzugt der vorhandene 3'-Überhang den Elongationsprozess am Zielsequenz-Unabhängigen-Reportermolekül behindern und es wird kein Signal erzeugt. Daher kann im Falle der SNP-Detektion das erste Nukleotid bevorzugt am 5'-Ende des zielspezifischen Sondenteils einer Mediatorsonde über der SNP-Position positioniert werden, um den spezifischen Zielsequenz-Unabhängigen-Reportermolekül-Typ zu aktivieren.

In einem weiteren Aspekt bezieht sich die Erfindung auf ein Kit umfassend:
- mindestens ein Zielsequenz-Unabhängiges-Reportermolekül eines ersten Typs umfassend mindestens eine Markierung,
- optional mindestens ein Zielsequenz-Unabhängiges-Reportermolekül eines zweiten Typs umfassend mindestens eine Markierung, welche bevorzugt im selben Detektionskanal oder dem entsprechenden Bereich eines Datenraums ein Signal emittiert, wie das mindestens eine Zielsequenz-Unabhängige-Reportermolekül eines ersten Typs,
- optional mindestens eine erste Mediatorsonde, deren Mediatorsequenz eine Affinität für das mindestens eine Zielsequenz-Unabhängige-Reportermolekül eines ersten Typs besitzt, und deren Oligonukleotidsequenz eine Affinität zu einer ersten Nukleinsäure-Zielsequenz aufweist,
- optional mindestens eine zweite Mediatorsonde, deren Mediatorsequenz eine Affinität für das mindestens eine Zielsequenz-Unabhängige-Reportermolekül eines zweiten Typs besitzt, und deren Oligonukleotidsequenz eine Affinität zu einer zweiten Nukleinsäure-Zielsequenz aufweist,
- optional mindestens ein Puffer,
- optional eine Polymerase
- optional eine Reverse-Transkriptase
- optional mindestens ein PCR-Primer.

In einer Ausführungsform ist das erfindungsgemäße Kit für die Durchführung des erfindungsgemäßen Verfahrens geeignet oder konfiguriert.

In Ausführungsformen kann ein erfindungsgemäßes Kit mehr als zwei unterschiedliche Typen von Zielsequenz-Unabhängigen-Reportermolekülen umfassen und optional auch mehr als eine erste und eine zweite Mediatorsonde, deren Mediatorsequenz eine Affinität zu den jeweiligen Zielsequenz-Unabhängigen-Reportermolekülen eines ersten oder zweiten oder weiteren Typs besitzen, und deren Oligonukleotidsequenz entsprechend jeweils eine Affinität zu einer ersten oder einer zweiten oder weiteren Nukleinsäure-Zielsequenzen aufweisen, sodass mehr als zwei unterschiedliche Nukleinsäure-Zielsequenzen spezifisch detektiert werden können.

Die für einen Aspekt der Erfindung beschriebenen Ausführungsformen können auch Ausführungsformen von jedem der anderen Aspekte der vorliegenden Erfindung sein. Dementsprechend können für das erfindungsgemäße Verfahren beschriebene Ausführungsformen auch Ausführungsformen des erfindungsgemäßen Kits sein. Außerdem kann jede hierin beschriebene Ausführungsform auch Merkmale jeder anderen Ausführungsform der Erfindung umfassen. Die verschiedenen Aspekte der Erfindung werden durch die gemeinsame und überraschende Entdeckung der unerwarteten vorteilhaften Wirkungen des vorliegenden Verfahrens, nämlich der optimierten gleichzeitigen PCR-Detektion von multiplen Zielsequenzen durch Zielsequenz-Unabhängige-Reportermoleküle vereint, profitieren davon, basieren darauf und/oder sind damit verbunden.

### DETAILLIERTE BESCHREIBUNG DER ERFINDUNG

Der Begriff "Zielsequenz-Unabhängiges-Reportermolekül" beschreibt ein Molekül oder Komplex aus mindestens einem DNA-Oligonukleotid zur Signalgenerierung während einer PCR in Gegenwart von DNA-Zielsequenzen. Vorzugsweise umfasst ein Zielsequenz-Unabhängiges-Reportermolekül mindestens eine Markierung, sowie mindestens einen Quencher.

In Ausführungsformen umfasst ein Zielsequenz-Unabhängiges-Reportermolekül ein oder mehrere Mediator-Bindestellen. Somit kann ein Zielsequenz-Unabhängiges-Reportermolekül mindestens 1, 2, 3, 4, 5, 6, 7, 8, 9, 10, 11, 12, 13, 14, 15, 16, 17, 18, 19, 20, 25, 30, 40 oder sogar 50 Mediator-Bindestellen umfassen. In bevorzugten Ausführungsformen umfasst ein Zielsequenz-Unabhängiges-Reportermolekül zwischen 1 und 10 Mediator-Bindestellen. Ein an eine Mediator-Bindestelle gebundener (aktivierter) Mediator oder Mediatorsequenz kann bei seiner Verlängerung durch eine (PCR) Polymerase zur Aktivierung bzw. dem Abbau, Verdau, (Ab)Spaltung oder Freisetzung eines oder mehrerer Markierungen, z.B. Quencher und/oder Fluorophore von einer oder mehrerer, vorzugsweise stromaufwärts (gen 5' Ende) am Reportermolekül gelegenen/lokalisierten Modifikation führen.

Im Kontext der vorliegenden Erfindung können Mediator-Nukleinsäuresequenzen sowohl als Mediatorsonde, als auch mit dem englischen Begriff "Mediator Probe" oder "Mediatorprobe" bezeichnet werden. Gemeint sind immer Nukleinsäure-Sonden, welche ein Signal zwischen einer zur Mediatorsonde homologen Ziel-Nukleinsäure und einem Zielsequenz-Unabhängige-Reportermolekülen übertragen. Während einer Nukleinsäure-Amplifikationsreaktion, z.B. PCR oder digitalen PCR-Reaktion, kann eine an der Ziel-Nukleinsäure vorwärts wandernde Polymerasen eine an die Ziel-Nukleinsäure gebundene Mediatorsonde spalten oder verdauen. Daraufhin wird eine Mediatorsequenz, oder auch einfach als "Mediator" bezeichnet, freigesetzt, welche an eine Mediatorbindestelle eines Zielsequenz-Unabhängigen-Reportermoleküls binden kann. Bindet eine Polymerase im Rahmen der PCR nun auch an das Reportermolekül und beginnt die gebundenen Mediatorsequenz zu verlängern, wird vorzugsweise eine Markierung aktiviert, z.B. vorzugsweise ein Quencher von einem Fluorophor getrennt, sodass ein Signal erzeugt wird und/oder eine Signaländerung bewirkt wird. In anderen Worten, hierin bezieht sich ein "Mediator" oder "Mediatorsequenz" auf ein Nukleinsäure-Oligonukleotid, welches durch die Polymerase entlang des Zielsequenz-Unabhängigen-Reportermoleküls verlängert werden kann. Eine "Mediatorsonde" beschreibt ein Nukleinsäure-Oligonukleotid, vorzugsweise ein DNA-Oligonukleotid, welches die Verbindung zwischen der Zielsequenz und dem Zielsequenz-Unabhängigen-Reportermolekül herstellt, indem es während einer PCR in Gegenwart einer DNA-Zielsequenz an diese bindet, durch die Exonuklease-Aktivität der Polymerase gespalten wird und eine Mediatorsequenz freisetzt, welche daraufhin an ein zugehöriges Zielsequenz-Unabhängiges-Reportermolekül bindet, zu welchem es eine Affinität besitzt.

Im Kontext der vorliegenden Erfindung beschreibt eine Affinität zu einer Nukleinsäure-Zielsequenz eine physikalische Anziehungskraft, welche zwei homologe Nukleinsäuresequenzen zueinander besitzen, und welche dazu führen kann, dass diese aneinander binden bzw. miteinander hybridisieren. Für eine Affinität oder erfolgreiche Hybridisierung zweier homologer Nukleinsäuresequenzen miteinander besteht in bevorzugten Ausführungsformen eine vollständige Homologie zwischen zwei Nukleinsäuresequenzen einer bestimmten Länge, wie z.B. zwischen einer Sondensequenz und einer Zielnukleinsäure Sequenz. In anderen Ausführungsformen können die homologen Sequenzen mindestens einen Mismatch zueinander enthalten.

Im Kontext der vorliegenden Erfindung wird ein Datenraum vorzugsweise durch einen Plot, ein Diagramm oder einen Graphen der unterschiedlichen Detektionskanäle erzeugt oder durch eine rein mathematische, Algorithmen- und/oder computerbasierte Auswertung, Vergleich oder Gegenüberstellung der unterschiedlichen Detektionskanäle, wobei die jeweiligen Fluoreszenzsignale der eingesetzten Zielsequenz-Unabhängigen-Reportermoleküle in diesem 1 - n dimensionalen Datenraum (je nach Anzahl der für die Auswertung benötigten Detektionskanäle) Datenpunkte generieren, welche sich zu Clustern (Gruppen, Ansammlungen) gruppieren können. Somit kann in Ausführungsformen ein "Datenraum" den Raum welcher in einem Plot, Diagramm oder Graph bevorzugt durch eine X-Achse und eine Y-Achse (zweidimensionaler Plot), und optional -bei einem dreidimensionalen Graph- zusätzlich durch eine Z-Achse, oder bei höheren Dimensionen zusätzlich durch eine weitere Achse erzeugt oder "aufgespannt" wird und in dem die darzustellenden Daten dargestellt werden. In Ausführungsformen in denen auf den Achsen eines Graphs oder Plots die, in den jeweiligen Detektionskanälen detektierten, Signale dargestellt werden - zum Beispiel die Signale des roten Detektionskanals auf der X-Achse, die Signale des grünen Detektionskanals auf der Y-Achse und die Signales des blauen Detektionskanals auf der Z-Achse des Plots - kann der Datenraum auch als "von den Detektionskanälen aufgespannt" bezeichnet werden. In weiteren Ausführungsformen wird ein Datenraum durch eine rein Algorithmus- und/oder computerbasierte Auswertung, Vergleich und/oder Gegenüberstellung der unterschiedlichen Detektionskanäle und/oder der darin detektierten Signale generiert und dabei vorzugsweise durch die analysierten Detektionskanäle "aufgespannt".

Durch die Anwendung des erfindungsgemäßen Verfahrens ist es in Ausführungsformen möglich, im höherdimensionalen Datenraum (>3 Dimensionen, bei mehr als drei Detektionskanälen) eine Datenklassifizierung durchzuführen. Durch das erfindungsgemäße Verfahren kann in Ausführungsformen die Anzahl an unterscheidbaren Zielsequenzen in einer digitalen PCR mindestens gegenüber den zur Verfügung stehenden Detektionskanälen verdoppelt werden, sofern dies nicht *per* se durch geräteseitige technische Limitationen, wie einem möglichen Crosstalk zwischen unterschiedlichen Detektionskanälen, eingeschränkt ist.

Im Kontext der vorliegenden Erfindung kann eine "Markierung" ein oder mehrere Fluorophore und/oder ein oder mehrere Quencher umfassen. Dementsprechend können im Kontext der Erfindung Reportermoleküle ein oder mehrere Fluorophore und/oder Quencher tragen bzw. umfassen. Die Nähe eines Fluorophors zum Quencher verhindert den Nachweis/Detektion seiner Fluoreszenz. In Ausführungsformen wird die Reporter-Quencher-Nähe unterbrochen, z.B. durch Freisetzung eines oder mehrerer Fluorophore und/oder Quencher, wobei dies in Ausführungsformen durch den teilweisen Abbau des Reportermoleküls, z.B. am 5' Ende, durch Hydrolyse durch die 5`-zu-3'-Exonuklease-Aktivität der PCR-Polymerase, die für die Amplifikationsreaktion verwendet wird, geschieht. Somit wird eine ungelöschte Emission von Fluoreszenz ermöglicht, die nach Anregung mit einem Laser nachgewiesen werden kann. In bevorzugten Ausführungsformen umfasst ein Zielsequenz-Unabhängiges-Reportermolekül mindestens ein Fluorophor und mindestens ein Quencher (diese sind bevorzugt also "paarweise" vorhanden), wobei der Quencher vorzugsweise das Fluorophor-Signal unterdrückt, bis eine Mediatorsequenz an das Reportermolekül bindet und vorzugsweise durch eine Polymerase im Rahmen einer Nukleinsäure-Amplifikationsreaktion verlängertwird. In Ausführungsformen wird durch die Polymerase eine räumliche Trennung, z.B. mindestens eines Fluorophors von mindestens einem Quencher, erreicht, sodass ein Signal generiert wird und detektiert werden kann. In Ausführungsformen, in denen mehrere Quencher-Fluorophor-Paare innerhalb eines Zielsequenz-Unabhängigen-Reportermolekül vorhanden sind, können die Paare jeweils auch in unterschiedlichen Lokalisationen zueinander angeordnet sein. Ein Zielsequenz-Unabhängiges-Reportermolekül kann eine oder mehrere Markierungen umfassen, so können zum Beispiel mindestens 1, 2, 3, 4, 5, 6, 7, 8, 9, 10, 11, 12, 13, 14, 15, 16, 17, 18, 19, oder 20 Markierungen, oder exakt 1, 2, 3, 4, 5, 6, 7, 8, 9, 10, 11, 12, 13, 14, 15, 16, 17, 18, 19, 20 oder sogar 25 Markierungen vorhanden sein. In manchen Ausführungsformen umfasst ein Zielsequenz-Unabhängiges-Reportermolekül 1, 2, 3 oder bis zu 5 Markierungen. In anderen Ausführungsformen umfasst ein Zielsequenz-Unabhängiges-Reportermolekül 1, 2, 3, 4, 5, oder sogar mehr als 5 Markierungen.

Erfindungsgemäß ist eine signalerzeugende Markierung bevorzugt ein Fluorophor oder anderer Farbstoff, welcher ein detektierbares Signal erzeugen kann. In manchen Ausführungsformen ist eine signalerzeugende Markierung eine elektroaktive oder magnetische Markierung. Im Kontext mancher Ausführungsformen sind die Begriffe "signalerzeugende Markierung" und "Markierung" äquivalent oder austauschbar.

Im Kontext der Erfindung umfasst ein Biomolekül vorzugsweise Nukleinsäuren.

Der Begriff "Nukleinsäure" bezieht sich auf Nukleinsäuremoleküle, einschließlich, ohne Einschränung, DNA, ssDNA, dsDNA, RNA, mRNA, tRNA, IncRNA, ncRNA, microRNA, siRNA, rRNA, sgRNA, piRNA, rmRNA, snRNA, snoRNA, scaRNA, gRNA oder virale RNA. Nukleinsäuresequenzen beziehen sich hierin auf eine aufeinanderfolgende Anordnung von Nukleotiden, wobei die Nukleotide durch ihre Nukleobasen in Guanin (G), Adenin (A), Cytosin (C) und Thymin (T) in DNA und Uracil (U) in RNA dargestellt werden. Eine Nukleinsäuresequenz kann sich hier auch auf die Sequenz aufeinanderfolgender Buchstaben bzw. Nukleobasen (bestehend aus G, A, C und T oder U) beziehen, die die tatsächliche Sequenz aufeinanderfolgender Nukleinsäuren in einem DNA- oder RNA-Strang darstellen. Diese Nukleinsäuresequenz kann unter Verwendung von DNA- oder RNA-Sequenzierung biochemisch und bioinformatisch identifiziert und charakterisiert werden oder durch komplementäre Nukleinsäure-Sonden (z.B. in Ausführungsformen hierin durch Mediatorsonden) spezifisch detektiert werden, z.B. im Rahmen einer PCR, real-time PCR oder Detektionsreaktion einer digitalen PCR. Eine Sequenzanalyse kann auch den Vergleich der erhaltenen Nukleinsäuresequenz oder eines dafür spezifischen Detektionssignals mit einer oder mehrerer Referenz-Nukleinsäuresequenzen und/oder mit den Detektionssignalen von Haushaltsgenen umfassen. Der Begriff Nukleotid kann mit "nt" abgekürzt werden. Der Begriff Basenpaar (zwei über Wasserstoffbrücken aneinander gebundene Nukleobasen) kann mit "bp" abgekürzt werden.

Eine "Zielsequenz" (hierin auch als "Target" bezeichnet) beschreibt im Kontext der Erfindung jede Nukleinsäuresequenz von Interesse, welche durch das erfindungsgemäße Verfahren nachgewiesen werden soll. Eine Zielsequenz kann vorzugsweise eine DNA, cDNA, cfDNA oder RNA Sequenz sein. Eine Zielsequenz kann ein Teil oder die gesamte Nukleinsäuresequenz einer Ziel-DNA darstellen. Eine Mediatorsonde umfasst vorzugsweise eine Sequenz, welche ganz oder teilweise komplementär zu der Nukleinsäuresequenz der Zielsequenz oder einem Teil davon ist. In manchen Ausführungsformen ist diese Sequenz der Mediatorsonde zu 100%, zu 99 %, 95%, 90% oder zu 80% komplementär zu der Zielsequenz. Eine Mediatorsonde kann in manchen Ausführungsformen eine oder mehrere Fehlpaarungen zu der Zielsequenz tolerieren und trotzdem an diese binden. In andere Ausführungsformen bindet die Mediatorsonde nur an eine Zielsequenz, wenn sie zu 100% mit der Zielsequenz übereinstimmt.

Der Begriff "Nukleinsäure-Amplifikationsreaktion" bezieht sich auf jedes Verfahren, das eine enzymatische Reaktion umfasst, die die Amplifikation von Nukleinsäuren ermöglicht. Eine bevorzugte Ausführungsform der Erfindung betrifft eine Polymerase-Kettenreaktion (PCR). Die "Polymerase-Kettenreaktion" ("PCR") ist die Goldstandard Methode, um schnell Millionen bis Milliarden Kopien (vollständige Kopien oder Teilkopien) einer bestimmten DNA-Probe herzustellen, wodurch die Amplifikation einer sehr kleinen DNA-Probe auf eine ausreichend große Menge ermöglicht wird. PCR amplifiziert eine bestimmte Region eines DNA-Strangs (die DNA-Zielsequenz), je nachdem, wo die verwendeten Primer binden, um die Amplifikationsreaktion zu starten. Fast alle PCR-Anwendungen verwenden ein hitzestabiles DNA-Polymerase-Enzym, wie z. B. Taq-Polymerase.

"Multiplexing" bedeutet im Allgemeinen, mehrere Zielsequenzen in einer Reaktion gleichzeitig nachzuweisen, ohne dass es nötig ist das Reaktionsgemisch für den Nachweis mehrerer Zielsequenzen weiter aufzuteilen. Bei einer "Multiplex-PCR" werden mehrere einzelne PCR-Reaktionen für verschiedene DNA-Sequenzen oder Gene unter identischen Bedingungen zu einem einzigen Reaktionsgemisch kombiniert. Indem mehrere Sequenzen gleichzeitig analysiert werden können, können Informationen aus einer einzigen PCR-Reaktion gewonnen werden, für die andernfalls ein Vielfaches an Reagenzien und mehr Zeit zur Durchführung erforderlich wären. Somit reduziert "multiplexing" den Kosten- und Zeitaufwand für die Durchführung einer PCR, da weniger Reagenzien pro Experiment verwendet, Experimente schneller durchgeführt und Ergebnisse schneller analysiert werden können. Da durch das Aufteilen eines Reaktionsgemisches eine nachzuweisende Zielsequenz auf eine Einzelnachweisreaktion aufgeteilt werden kann, in welcher sie nicht nachgewiesen werden würde, sind multiplex Nachweise sensitiver und sind daher insbesondere in onkologischen Diagnosen sehr vielversprechende Nachweismethoden. Da auch Pipettierfehler bei einer Reaktion gegenüber mehreren minimiert werden können, kann der Einsatz einer Multiplex-PCR auch die Präzision gegenüber Einzelassays verbessern.

Quantitative PCR ("qPCR") oder"real-time PCR" ("Echtzeit-PCR"), stellt eine spezifische Form der PCR dar und ist eine Standardmethode zum Nachweis und zur Quantifizierung einer spezifischen Zielsequenz oder zur Quantifizierung des Genexpressionsniveaus in einer Probe in Echtzeit. Bei der qPCR werden fluoreszenzmarkierte Sonden oder Nukleinsäuren (z.B. Mediatorsonden) in der PCR-Reaktion hybridisiert und in Ausführungsformen von der PCR-Polymerase eingebaut bzw. verlängert sobald sie an eine komplementäre Sequenz binden (z.B. eine Zielsequenz), wobei in Ausführungsformen das Vorhandensein und die Amplifikation von Zielsequenzen nach oder während eines jeden PCR-Zyklus in Echtzeit überwacht wird. Eine real-time PCR ermöglicht es, den Fortschritt einer laufenden Amplifikationsreaktion während ihres Auftretens (d. h. in Echtzeit) zu überwachen. Daten werden daher während der PCR-Reaktion gesammelt und nicht am Endpunkt wie bei der herkömmlichen PCR. Die Messung der Reaktionskinetik in den frühen Phasen der PCR bietet deutliche Vorteile gegenüber dem herkömmlichen PCR-Nachweis. In Ausführungsformen der real-time PCR werden Reaktionen durch den Zeitpunkt während des Zyklus charakterisiert, an dem die Amplifikation eines Ziels erstmals nachgewiesen wird, und nicht durch die Menge des Ziels, die sich nach einer festgelegten Anzahl von Zyklen angesammelt hat, wie bei einer klassischen PCR. Je höher die Start-Kopienanzahl des Nukleinsäure-Targets ist, desto eher wird eine signifikante Zunahme der Fluoreszenz beobachtet. Die real-time PCR ermöglicht eine Analyse mittels optischer Signale, die zum Nachweis eines spezifischen PCR-Produkts (der Zielsequenz), wobei spezifische Fluorochrome bzw. Fluorophore verwendet werden. Eine Zunahme des DNA-Produkts während einer PCR führt daher zu einer Zunahme der bei jedem Zyklus gemessenen Fluoreszenzintensität. Unter Verwendung verschiedenfarbiger Markierungen können fluoreszierende Sonden in Multiplex-Assays zum Überwachen mehrerer Zielsequenzen verwendet werden.

Während die Real-time qPCR davon abhängig ist, dass in jedem Amplifikationszyklus die relative Menge der Zielnukleinsäure bestimmt wird, ermöglicht die "digitale PCR" hingegen eine Bestimmung der absoluten Menge der Zielnukleinsäure auf Basis der Poisson-Statistik, welche im Anschluss an eine Endpunkt-PCR-Amplifikation für die Berechnung der Zielnukleinsäure-Menge angewandt wird. Die Schritte vor der Amplifikation sind üblicherweise vergleichbar oder ähnlich zwischen digitaler PCR und qPCR. Jedoch werden in der qPCR vorzugsweise alle Nukleinsäuremoleküle gepoolt und anschließend amplifiziert und analysiert, während in der digitalen PCR die Nukleinsäuremoleküle vorzugsweise bestmöglich auf Einzelpartitionen (z.B. Emulsions-Tropfen, Kavitäten oder Gel-Beads) aufgeteilt werden, wodurch die PCR in jeder Partition als Einzelreaktion abläuft (im Fall von Emulsions-Tropfen, wird diese Reaktion auch oft als droplet PCR oder digital droplet PCR bezeichnet) und eine separate Analyse jeder Partition ermöglicht. Die zufällige Aufteilung der Nukleinsäuremoleküle in einzelne Partitionen erfolgt bei der digitalen PCR gemäß der Poisson-Verteilung. Bei der Analyse der digitalen PCR wird dann die Poisson-Statistik angewandt, um die durchschnittliche Anzahl an Nukleinsäuremolekülen pro Partition (keines, eines oder mehrere) zu bestimmen. Die statistische Poisson-Analyse der Anzahl positiver und negativer Reaktionen liefert eine präzise absolute Quantifizierung der Target-Sequenz.

Die Recombinase Polymerase Amplification ("RPA", engl. für ,Rekombinase-Polymerase-Amplifikation') ist eine Methode zur Amplifikation (Vervielfältigung) von DNA und ist eine Variante der isothermalen DNA-Amplifikation. RPA wird in der Regel unter der Verwendung einer Rekombinase (Einzelstrang-bindendes Protein) und einer strangversetzenden DNA-Polymerase durchgeführt, wobei die Rekombinase die Primer-Bindung erhöht. Wenn eine strangversetzende Polymerase verwendet wird, ist es möglich die Reaktion bei 37 bis 42 °C oder sogar bei Raumtemperatur durchzuführen. Durch Zugabe eines reversen Transkriptase-Enzyms zu einer RPA-Reaktion kann eine reverse Transkription durchgeführt werden. In verschiedenen Varianten der RPA kann die entstehende DNA eine RPA-Reaktion optional zusätzlich quantifiziert werden (ähnlich zur qPCR) und/oder mehrere DNA-Sequenzen können in einem Multiplex-Verfahren parallel amplifiziert werden.

Der Begriff "loop-mediated isothermal amplification" oder "LAMP" (englisch für "Schleifen-vermittelte isothermale Amplifikation") bezeichnet eine Methode zur Amplifikation von DNA, wobei auch die LAMP eine Variante der isothermen DNA-Amplifikation darstellt. Eine isotherme Amplifikationsreaktion erfolgt in der Regel bei einer konstanten Temperatur. Diese Eigenschaft unterscheidet die LAMP von der PCR bei der die Reaktion mit einer Reihe von alternierenden Temperaturschritten oder Zyklen erfolgt. In der LAMP wird üblicherweise eine strangversetzende DNA-Polymerase eingesetzt. Bei einer LAMP-Reaktion können, zum Beispiel, vier bis sechs Primer an sechs bis acht DNA-Sequenzen binden, was ein besonderes Primerdesign erfordert. Im Rahmen einer LAMP-Reaktion kann durch Zugabe einer reversen Transkriptase eine reverse Transkription durchgeführt werden. Zusätzlich kann die in einer LAMP-Reaktion entstehende DNA quantifiziert werden. Es ist auch möglich mehrere DNA-Sequenzen parallel in einer Multiplex-LAMP-Reaktion nachzuweisen.

Im Kontext der Erfindung beschreibt eine "Signaländerung" eine Fluoreszenzsignaländerung. Diese Signaländerung ist vorzugsweise eine signifikante, differenzierbare und/oder charakteristische Änderung des Fluoreszenzsignals, welche sich deutlich von potentiellen Basis- oder Hintergrundsignalen bzw. Grund- oder Hintergrundrauschen abgrenzt bzw. unterscheidet. Dem Fachmann ist bewusst, dass unter manchen Versuchsbedingungen im Rahmen von Fluoreszenzdetektionen unspezifische Fluoreszenz-Basissignale bzw. Grund- oder Hintergrundrauschen durch Fluorophore auftreten können. Daher beschreibt eine Signaländerung im Kontext der Erfindung vorzugsweise eine signifikante, differenzierbare und/oder charakteristische Änderung des Fluoreszenzsignals, und kein Fluoreszenz-Basis- oder Hintergrundsignal bzw. Grund- oder Hintergrundrauschen. Diese Signaländerung kann in bevorzugten Ausführungsformen eine Zunahme der Fluoreszenzintensität, in anderen Worten eine Zunahme des Fluoreszenzsignals bedeuten. In manchen Ausführungsformen ist eine Signaländerung die Abnahme des Fluoreszenzsignals. Die Zunahme eines Fluoreszenzsignals ist vorzugsweise dadurch bedingt, dass durch eine Amplifikationsreaktion die Anzahl der Zielsequenz-Amplifikate steigt und dadurch die Aktivierung zugehöriger Signalkomplexe. Entsprechend steigt die Anzahl der jeweils daraus resultierenden (Ab-)Spaltungen, Verdaue und/oder Trennungen der jeweiligen Signaloligos von ihrer Bindestelle auf den zugehörigen Basissträngen, wodurch je mindestens ein Fluorophor freigesetzt und/oder von seinem Quencher getrennt wird (d.h. der Abstand von Quencher und Fluorophor sich derart vergrößert, dass das Fluoreszenzsignal nicht mehr durch den Quencher gelöscht wird). Eine Zunahme (Vergrößerung der Anzahl) der freigesetzten und/oder nicht-gelöschten Fluorophore führt somit zu einer Zunahme des Fluoreszenzsignals, welches hierin jeweils für eine Zielsequenz spezifisch und indikativ ist. Je mehr Zielsequenzen somit vorhanden sind und durch Mediatorsonden in einer PCR Reaktion gebunden werden, umso mehr steigt das Fluoreszenzsignal. Vorzugsweise ist das Fluoreszenzsignal proportional oder annährend proportional zu der Menge der korrespondierenden Zielsequenz, für welche das Fluorophor-Signal (z.B. seine Farbe) spezifisch/charakteristisch ist. Da im Rahmen einer digitalen oder "droplet" PCR bevorzugt nur eine Zielsequenz je Reaktionsraum (z.B. Partition, Emulsions-Tropfen) vorhanden ist, steigt das Signal zunehmend mit der Anzahl der Zielsequenz-Amplifikate je Reaktionsraum bis zum Erreichen eines Signalplateaus, welches durch die maximale Fluoreszenzintensität der für den jeweiligen Nachweis eingesetzten Reportermoleküle vorgegeben ist. In bevorzugten Ausführungsformen liegt idealerweise eine gleichmäßige Verteilung von max. 1 Zielsequenz je Reaktionsraum (z.B. Partition, Emulsions-Tropfen) bei beginnender Amplifikation vor, sodass bei einer ähnlichen Amplifikationseffizienz und ähnlicher maximaler Fluoreszenzintensität der Nachweismoleküle in allen Reaktionsräumen (die eine Zielsequenz enthalten) das spezifische Signal für den Nachweis einer identischen Zielsequenz in unterschiedlichen Reaktionsräumen mit vergleichbarer Höhe/Stärke/Intensität bei der Auslese mittels digitaler oder "droplet" PCR entsteht, welches vorzugsweise indikativ für das Vorhandensein und/oder die Anzahl der vorhandenen Zielsequenz-Amplifikate in jedem Reaktionsraum ist. In Ausführungsformen kann die, vorzugsweise für eine Zielsequenz spezifische, Intensität/Stärke einer jeweiligen Markierung sowie die maximal erreichbare Signalstärke/Intensität von der Anzahl der Markierungen je Signaloligo und Signalkomplex und/oder der Art der Markierung (z.B. Art der Fluorophore und/oder Quencher) abhängen.

"Fluorophor" (oder Fluorochrom, ähnlich einem Chromophor) ist eine fluoreszierende chemische Verbindung, die bei Lichtanregung Licht wieder emittieren kann. Fluorophore zur Verwendung als Markierungen bei der Konstruktion markierter Sonden der Erfindung umfassen, ohne Anspruch auf Vollständigkeit zu erheben, Rhodamin und Derivate, wie Texas Red, Fluorescein und Derivate, wie 5-Brommethylfluorescein, Lucifer Yellow, IAEDANS, 7-Me2N-Cumarin -4-Acetat, 7-OH-4-CH3-Cumarin-3-Acetat, Monobrombiman, Pyrentrisulfonate wie Cascade Blue und Monobromtrimethyl-Ammoniobiman, 7-NH2-4CH3-25-Cumarin-3-Acetat (AMCA), FAM, TET, CAL Fluor Gold 540, JOE, VIC, Quasar 570, CAL Fluor Orange 560, Cy3, NED, Oyster 556, TMR, CAL Fluor Rot 590, HEX, ROX, LC Rot 610, CAL Fluor Rot 610, Texas Rot, LC Rot 610, CAL Fluor Rot 610, LC Rot 640, CAL Fluor Rot 635, Cy5, LC Rot 670, Quasar 670, Oyster 645, LC Rot 705, Cy5.5, BODIPY FL, Rhodamine Green, Oregon Green 30 488, Oregon Green 514, Cal Gold, BODIPY R6Gj, Yakima Yellow, Cal Orange, BODIPY TMR-X, JOE, HEX, Quasar-570 /Cy3, TAMRA, Rhodamine Red-X, Redmond Red, BODIPY 581/591, Cy3.5, Cal Red/Texas Red, BODIPY TR-X , BODIPY 630/665-X, Quasar-670/Cy5, Pulsar-650, Dy490, Atto-488, Atto532, Atto-Rho-6G, Dy590, Atto-Rho101, Cy5, Dy-636, Atto-647N, Cy5.5, Dy682, Atto-680, BMN-488, BMN-505, BMN-536, BMN-562,

"Quenching" bezieht sich auf jeden Prozess, der die Fluoreszenzintensität einer gegebenen Substanz verringert. Quenchen ("Löschen") ist die Grundlage für Förster-Resonanzenergietransfer (FRET)-Assays oder Statische- bzw. Kontaktquenching-Assays oder einer Kombination aus beiden. FRET ist ein dynamischer Löschmechanismus, da die Energieübertragung stattfindet, während sich der Donor im angeregten Zustand befindet. Beim Kontaktquenchen ist eine unmittelbare räumliche Nähe in Form eines physischen Kontakts von Donor und Quencher notwendig. Ein "Quencher" ist ein Molekül, das die vom Fluorophor emittierte Fluoreszenz löscht, wenn dieses durch die Lichtquelle eines PCR-Cyclers oder Detektionsgerätes angeregt wird. Quencher zur Verwendung als Markierungen bei der Konstruktion markierter Signaloligos und/oder Basisstränge der Erfindung umfassen, ohne Anspruch auf Vollständigkeit zu erheben, DDQ-I, lowa Black, lowa Black FQ, QSY-9, BHQ-1, QSY-7, BHQ-2, DDQ-II, 22 Eclipse, lowa Schwarz RQ, QSY-21, BHQ-3 Dabcyl,, QSY-35, BHQ-0, ElleQuencher, BMN-Q1, BMN-Q2, BMN-Q60, BMN-Q-535, BMN-Q590, BMN-Q620, BMN-Q650. Der Fachmann kennt geeignete Reporter-Quencher-Paare und weiß, welche für eine jeweilige Anwendung auszuwählen sind.

### AUSFÜHRUNGSBEISPIELE

Die Erfindung wird durch die folgenden Beispiele weiter beschrieben. Diese sollen den Umfang der Erfindung nicht einschränken, sondern stellen bevorzugte Ausführungsformen verschiedener Aspekte der Erfindung dar, welche zur Veranschaulichung der hierin beschriebenen Erfindung bereitgestellt werden.

### Methoden

Im den folgenden Ausführungsbeispielen wurden PCR-Amplifikationen in Form von digitaler PCR durchgeführt. Digitale PCR bezeichnet eine PCR-Amplifikationsreaktion in welcher einzelne Nukleinsäuremoleküle vorzugsweise auf einzelne Partitionen (z.B. Emulsions-Tropfen, Kavitäten oder Gel-Beads) aufgeteilt werden, wodurch die PCR in jeder Partition als Einzelreaktion abläuft (im Fall von Emulsions-Tropfen, wird diese Reaktion auch oft als droplet PCR oder digital droplet PCR bezeichnet) und eine separate Analyse jeder Partition ermöglicht. Diese Analyse erfolgt vorzugsweise durch Fluoreszenzanalyse (z.B. durch Laser-Anregung eines Fluorophors und der Detektion von dessen emittierter Fluoreszenz) jeder einzelnen Partition. Befindet sich eine Zielsequenz in einer Partition, und wird diese durch das erfindungsgemäße Verfahren detektiert, wird dies vorzugsweise durch ein spezifisches Fluoreszenzsignal angezeigt.

Für die aufgeführten Ausführungsbeispiele wurde das Naica System von Stilla Technologies verwendet, im ersten Schritt wurde mit der Geode die Tropfen generiert und thermisch prozessiert. Das verwendet dPCR-Protokoll war 5 min bei 95°C gefolgt von 45 Zyklen mit 15 sec bei 95°C und 60 sec bei 58°C. Für die Auswertung der Tropfen wurde der Prism 3-Reader, sowie die dazu entwickelte CrystalMiner Software (Version 3.1.6.3) verwendet.

Für alle Reaktionen wurde einfach konzentrierter PerfeCTa^{®} MultiPlex qPCR ToughMix^{®} mit 50 nM Alexa Fluor 488 als Hintergrundfluorophor verwendet. In Duplex- und Vierplex-Reaktionen wurden für die jeweiligen Zielsequenzen 0,5 µM Vorwärts- und 0,25 µM Rückwärtsprimer, 1,2 µM Mediatorsonden und 0,6 µM Zielsequenz-Unabhängige-Reporter verwendet. Die Konzentration der Zielsequenz-Unabhängige-Reporter wurde verringert auf 0,4 µM für die Sechsplex-Reaktion.

### Sequenzen:

**Tabelle 1: Primer**

| SEQ ID | Name | Sequenz (5'-3') |
|---|---|---|
| 1 | KRAS_vorwärts | GGCCTGCTGAAAATGACT |
| 2 | KRAS_rückwärts | ACAAAATGATTCTGAATTAGCTGTA |
| 3 | BRAF_vorwärts | GACCCACTCCATCGAGATTTC |
| 4 | BRAF_rückwärts | GCTTGCTCTGATAGGAAAATGAG |

**Tabelle 2: Mediatorsonden**

| SEQ ID | Name | Sequenz (5'-3') |
|---|---|---|
| 5 | MP_ZUR02_KRAS_WT | ctccagttcggtCCAGCTCCAACTACCACAAGTTTATATTCAG |
| 6 | MP_ZUR04_KRAS_G12A | gatacagggtccaCTGGCGTAGGCAAGAGTGCCTTGACG |
| 7 | MP_ZUR05_KRAS_G12D | atgtcccaggtgcATGGCGTAGGCAAGAGTGCCTTGACGAT |
| 8 | MP_ZUR06_KRAS_G12V | aggtaggctcacTTGGCGTAGGCAAGAGTGCCTTGACGAT |
| 9 | MP_ZUR07_BRAF_V600E | acatgctcatgttgtgTCTGTAGCTAGACCAAAATCAC- CTATTTTTACTGTGAG |
| 10 | MP_ZUR08_BRAF_WT | gtgttcctcacatgctACTGTAGCTAGACCAAAATCAC- CTATTTTTACTGTGAG |

**Tabelle 3: Zielsequenz-Unabhängige-Reportermoleküle (Die Fluorophor-Modifikationen befinden sich jeweils intern an der ersten 3' Base der Stem-Loop-Struktur (nt22 (7) oder nt 20 (8); dargestellt als 7 oder 8); Quencher wurden an die erste Base der Stem-Loop-Struktur auf der 5'-Seite (nt 1) gebunden)**

| SEQ ID | Name | Sequenz (5'-3') | 5'-mod. Quencher | Interne Mod. Fluorophor |
|---|---|---|---|---|
| 11 | ZUR02 | GACCGGCCAAGACGCGCCGGT7TGTTCACTGAC- CGAACTGGAGCA | BMN-Q1 | **7**=dC-DY-530 |
| 12 | ZUR04 | GACCGCACTAGTAGATGCGGT7TGTCGTG- GACCCTGTATCGAGCA | BHQ-1 | 7=dC-BMN536 |
| 13 | ZUR05 | GACCGGCTAAGACGCGCCGGT7TGTTGCAC- CTGGGACATCGACTAT | BHQ-2 | 7=dC-Cy5 |
| 14 | ZUR06 | GACGCGTAGTACAGAACGCGT7TGTTCAG- TGAGCCTACCTGCCTTC | BHQ-2 | 7=dC-Atto 647N |
| 15 | ZUR07 | ATGCCGATTAGATGCGGCAT8TCGATCACACAACA TGAGCATGTGTAC | BHQ-1 | 8=dT-FAM |
| 16 | ZUR08 | ATCCGCCAAGACGCGCGGAT8TAGCATGTGAG- GAACACGATGACAC | BMN-Q1 | 8=dT-Atto 488 |

**Tabelle 4: Zielsequenzen (Primer-Hybridisierungsregionen sind durch Unterstreichung dargestellt; Punktmutationen/SNPs sind in Fettschrift dargestellt)**

| SEQ ID | Name | Sequenz (5'-3') |
|---|---|---|
| 17 | BRAF WT | CAAACTGATGGGACCCACTCCATCGAGATTTCACTGTAGCTAGAC- CAAAATCAC- CTATTTTTACTGTGAGGTCTTCATGAAGAAATATATCTGAGGTGTAGTAAGT AAAGGAAAACAGTAGATCTCATTTTCCTATCAGAGCAAGCATTATGAAGAG- TTT |
| 18 | BRAF V600E | CAAACTGATGGGACCCACTCCATCGAGATTTCTCTGTAGCTAGAC- CAAAATCAC- CTATTTTTACTGTGAGGTCTTCATGAAGAAATATATCTGAGGTGTAGTAAGT AAAGGAAAACAGTAGATCTCATTTTCCTATCAGAGCAAGCATTATGAAGAG- TTT |
| 19 | KRAS WT | CATTTTCATTATTTTTATTATAAGGCCTGCTGAAAATGACTGAATATAAACTT- GTGGTAGTT- GGAGCTGGTGGCGTAGGCAAGAGTGCCTTGACGATACAGCTAATTCAGAA TCATTTTGTGGACGAATAT |
| 20 | KRAS G12A | CATTTTCATTATTTTTATTATAAGGCCTGCTGAAAATGACTGAATATAAACTT- GTGGTAGTT- GGAGCTGCTGGCGTAGGCAAGAGTGCCTTGACGATACAGCTAATTCAGAA TCATTTTGTGGACGAATAT |
| 21 | KRAS G12D | CATTTTCATTATTTTTATTATAAGGCCTGCTGAAAATGACTGAATATAAACTT- GTGGTAGTT- GGAGCTGATGGCGTAGGCAAGAGTGCCTTGACGATACAGCTAATTCAGAA TCATTTTGTGGACGAATAT |
| 22 | KRAS G12V | CATTTTCATTATTTTTATTATAAGGCCTGCTGAAAATGACTGAATATAAACTT- GTGGTAGTT- GGAGCTGTTGGCGTAGGCAAGAGTGCCTTGACGATACAGCTAATTCAGAAT CATTTTGTGGACGAATAT |

### Ausführungsbeispiel 1

Im vorliegenden Beispiel wird ein Signal generiert, indem eine Mediatorsonde (Mediator Probe) an eine Nukleinsäure-Zielsequenz bindet und im Rahmen einer PCR-Reaktion von einer Polymerase, welche eine Nukleinsäuresequenz amplifiziert, verdaut oder gespalten wird. Somit wird der Mediator, oder auch Mediatorsequenz genannt, freigesetzt und kann an ein Zielsequenz-Unabhängiges-Reportermolekül binden. Wird der gebundene Mediator nun im Rahmen der PCR-Reaktion als Primer oder Start-Oligonukleotid für eine Amplifikation von einer Polymerase erkannt, durch die Polymerase gebunden und verlängert, erfolgt eine Abtrennung oder Verdrängung eines "downstream" (stromabwärts der Amplifikationsrichtung) von der Mediatorbindestelle lokalisierten Quenchers oder Fluorophors, sodass ein Signal durch einen - zuvor durch Kontakt-Quenching gelöschten - Fluorophor erzeugt wird. Dieses Signal ist charakteristisch für die Nukleinsäure-Zielsequenz, an welche die Mediatorsonde (Mediator Probe) zuvor gebunden hat. Das erfindungsgemäße Verfahren ermöglicht ein (direktes) monochromes Multiplexing in der digitalen PCR durch Einsatz von mindestens zwei Zielsequenz-Unabhängigen-Reportermolekülen (siehe z.B. "Zielsequenz-Unabhängiger-Reporter" in Figur 1) mit jeweils unterschiedlichen Fluorophor-Modifizierungen (siehe z.B. Figur 1, darin dargestellt als Kreis oder Dreieck) mit einem Emissionsmaximum in einem (z.B. im roten) Detektionskanal in einer Konfiguration, welche eine Signalidentifikation ermöglicht. In Figur 1 sind unterschiedliche Signalstärken der Fluoreszenz durch unterschiedlich große Kreise dargestellt. Durch den Einsatz Zielsequenz-Unabhängiger-Reportermoleküle kann im Gegensatz zu Zielsequenz spezifischen Taqman-Sonden des Standes der Technik effizienter ein Kontakt-Quenching eingestellt werden, wodurch die Unterschiede in der Signalstärke unterschiedlicher Fluorophore im selben Detektionskanal verstärkt oder leichter modelliert werden können.

Zum (direkten) Multiplexen, also dem gleichzeitigen Nachweis verschiedener Zielsequenzen in einer zu analysierenden Probe und/oder in einer Reaktion sind im bisherigem Stand der Technik meist mehrere optische Kanäle, weitere Prozessschritte oder eine aufwendige Konzentrationsabstimmung oder Modifikation der Reportermoleküle erforderlich. Im Gegensatz hierzu gibt das erfindungsgemäßes Verfahren die Möglichkeit, verschiedene Zielsequenzen in einer Probe im gleichen Detektionskanal zu detektieren ohne durch die vorab aufgeführten Punkte limitiert zu sein. Der Einsatz von Zielsequenz-unabhängigen Reportermolekülen ermöglicht zudem die Wiederverwendung optimierter Designs für verschiedene Zielsequenzen und macht deren Anwendung entsprechend effizienter. Hierbei ermöglichen es, z.B. verschiedene Intensitätskombinationen und/oder verschiedene Fluorophor-Farben der jeweiligen Markierungen, vorzugsweise unterschiedliche verlängerte Mediatorsequenzen und somit unterschiedliche nachgewiesene Zielsequenzen zu kodieren und zu differenzieren.

In Ausführungsformen können über verschiedene Fluoreszenzstärken und/oder Fluoreszenzfarben mehrere Zielsequenzen gleichzeitig nachgewiesen werden, indem verschiedene Zielsequenz-Unabhängige-Reportermoleküle eingesetzt werden. Hierbei besitzen diese Zielsequenz-Unabhängigen-Reportermoleküle jeweils unterschiedliche Mediatorbindestellen mit Fluoreszenz- und/ oder Quencher-Markierungen unterschiedlicher Signalintensität und/oder Farbe/Emissionsspektrum. Bevorzugt werden jedoch Zielsequenz-Unabhängige-Reportermoleküle eingesetzt von denen jeweils immer mindestens zwei Reportermoleküle Signale erzeugen können, welche im gleichen Detektionskanal gemessen/detektiert werden können.

Durch die Erzeugung von Signalen mit unterschiedlichen Signalstärken, sind diese so in einem Fluoreszenzkanal unterscheidbar. Diese Ausführungsformen bieten insbesondere in der digitalen PCR erhebliche Vorteile um unterschiedliche Signalcluster unterscheidbar zu machen und stellen eine Verbesserung des Standes der Technik dar.

### Ausführungsbeispiel 2

Ein Beispiel in welchem zwei Zielsequenz-Unabhängigen-Reportermoleküle mit jeweils unterschiedlicher Fluorophor und Quencher-Anzahlen und einem Emissionsmaximum im roten Detektionskanal verwendet werden ist ein weiteres Beispiel für (direktes) monochromes Multiplexing gemäß der vorliegenden Erfindung. In diesem Beispiel werden während der digitalen PCR jeweils unterschiedliche Fluoreszenz-Signalstärken durch unterschiedliche Anzahlen an Fluorophoren generiert und ermöglichen so eine Signalidentifikation. Durch den Einsatz Zielsequenz-Unabhängiger-Reportermoleküle kann, z.B. im Gegensatz zu Zielsequenz spezifischen Taqman-Sonden, effizienter eine symmetrische Mehrfachmodifizierung an Fluorophoren und Quenchern genutzt werden, wodurch die Unterschiede in der Signalstärke unterschiedlicher Fluorophore im selben Detektionskanal verstärkt werden.

### Ausführungsbeispiel 3

Bei einer Probenanalyse wurde ein (direktes) monochromes Multiplexing durch den Einsatz von 2 Zielsequenz-Unabhängigen-Reportermolekülen mit unterschiedlichen Fluorophor-Markierungen im roten Detektionskanal durchgeführt. Hierbei wurden die zwei unterschiedlichen Zielsequenz-Unabhängigen-Reportermoleküle entweder mit Cy5 oder mit Atto-647N markiert. Die Analyse umfasste ein Amplifikationsreaktion im Rahmen einer digitalen PCR ("Mediator Probe" / Mediatorsonden-PCR) im Stilla Naica System, wobei die erhaltenen Ergebnisse in mehreren Dimensionen eines Datenraums graphisch dargestellt werden können.

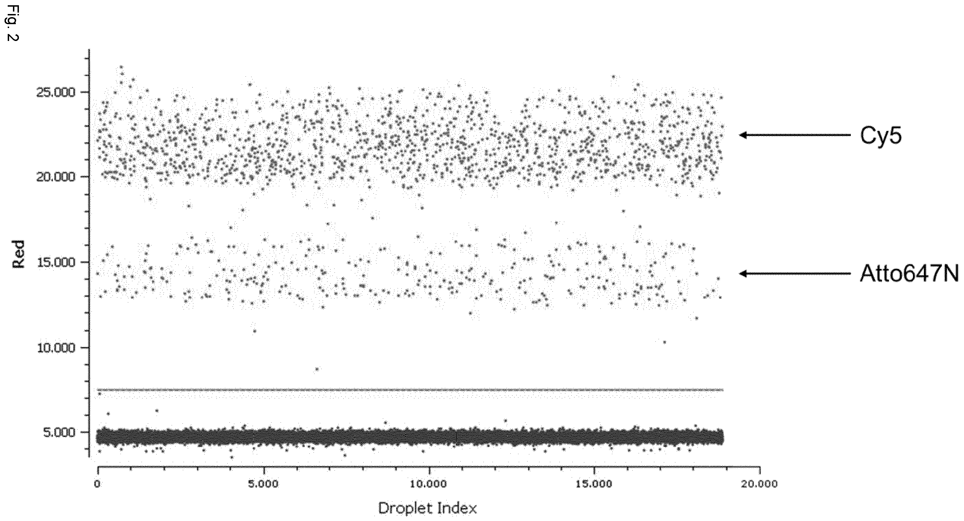

Figur 2 zeigt eine graphische Auswertung und Darstellung der digitalen PCR-Ergebnisse in Form eines 1-D (eindimensionalen) Plots.

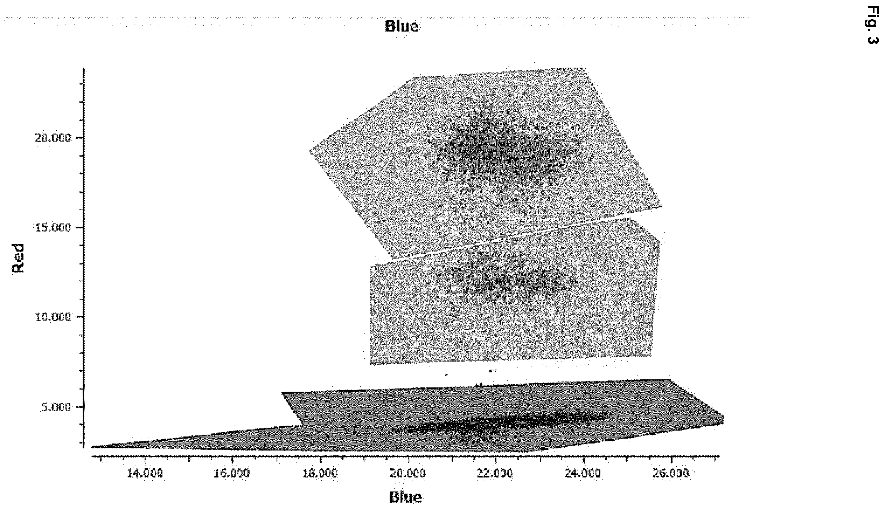

Figur 3 zeigt eine graphische Auswertung und Darstellung der digitalen PCR-Ergebnisse in Form eines 2-D (zweidimensionalen) Plots. Hierbei wurden die spezifischen Signale der Reporterkomplexe/Reportermoleküle im roten Kanal ("red", Y-Achse) dem Hintergrundsignal (Hintergrundrauschen) im bauen Detektionskanal ("blue", X-Achse) in einem zweidimensionalen Datenraum gegenübergestellt. Die zwei unterschiedlichen Signale der Reporterkomplexe/Reportermoleküle sind als distinkte Signalpopulationen im zweidimensionalen Datenraum gut voneinander, und vom Hintergrundrauschen (im blauen Detektionskanal) differenzierbar.

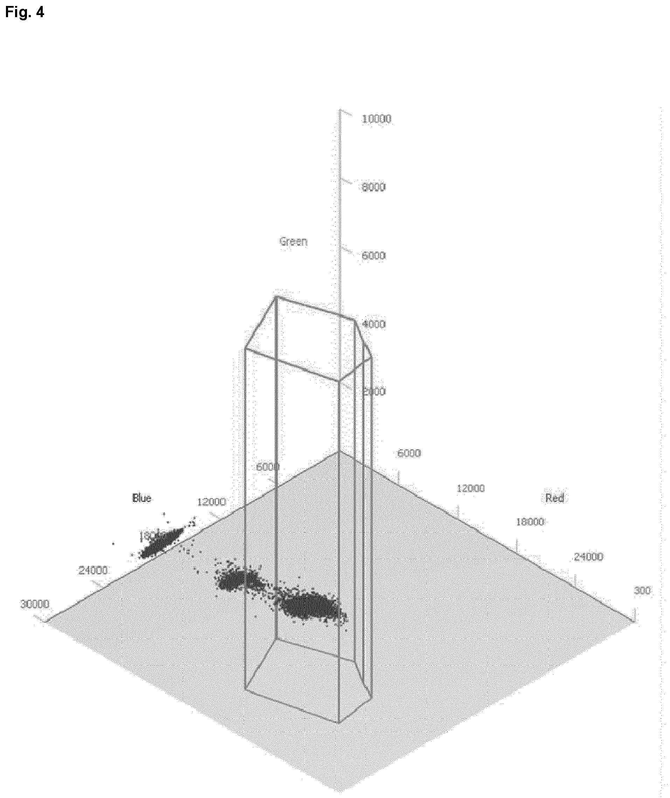

Figur 4 zeigt eine graphische Auswertung und Darstellung der digitalen PCR-Ergebnisse in Form eines 3-D (dreidimensionalen) Plots. Hierbei wurden die spezifischen Signale der Reporterkomplexe/Reportermoleküle im roten Kanal ("red", Y-Achse) sowohl dem Hintergrundsignal im bauen Detektionskanal ("blue", X-Achse) als auch den Signalen einer Negativkontrolle (Probe ohne Zielgen) im grünen Kanal ("green", Z-Achse) in einem dreidimensionalen Datenraum gegenübergestellt. Auch in dieser Ausführungsform sind die zwei unterschiedlichen Signale der Reporterkomplexe/Reportermoleküle als distinkte Signalpopulationen im dreidimensionalen Datenraum gut voneinander, und vom Hintergrundrauschen und einem - hier nicht detektierten, aber in anderen Analysen potentiell vorhandenen - Signal der Negativkontrolle differenzierbar.

### Ausführungsbeispiel 4

Im vorliegenden Experiment erfolgt in einer digitalen PCR-Reaktion unter Verwendung von Mediatorsonden (Mediator Probes) durch den Einsatz von vier verschiedenen Typen von Zielsequenz-Unabhängigen-Reportermolekülen (ZUR1-ZUR4) mit unterschiedlichen Fluoreszenzmarkierungen ein (direktes) monochromes Multiplexing in zwei Detektionskanälen (z.B. roter und grüner Kanal). Bei einer graphischen Darstellung der während der digitalen PCR-Reaktion detektierten Signale, welche durch die verschiedenen Typen von Reportermolekülen generiert werden, können diese in Form von Signalpopulationen im Datenraum (der Raum der durch eine X-Achse und eine Y-Achse generiert oder aufgespannt wird und in dem die Daten graphisch dargestellt werden) dargestellt werden.

Figur 5 zeigt ein theoretisches Schema der Darstellung einer Analyse von Signalen von einem Beispiel mit vier unterschiedlichen Reportermolekülen (ZUR1-ZUR4) wobei unterschiedliche Floreszenz-Signalstärken durch unterschiedlich große Kreise und Positionen im Datenraum dargestellt sind. Der Datenraum wird in Figur 5 durch die X-Achse, auf welcher die Intensität von detektierten Signalen im roten Detektionskanal (ZUR4 und ZUR2) dargestellt ist, und der Y-Achse, auf welcher die detektierte Signalintensität im grünen Detektionskanal (ZUR3 und ZUR1) dargestellt ist, aufgespannt. Die Signalpopulationen im Datenraum sind klar einem der vier Typen der aktivierten Zielsequenz-Unabhängigen-Reportermolekülen zuzuordnen und könnten daher im Gegensatz zu Zielsequenz-spezifischen Reportermolekülen (z.B. Taqman-Sonden) effizient zum Nachweis unterschiedlicherZielsequenz-Panels (Zielsequenzzusammenstellungen oder-kombinationen) genutzt werden.

### Ausführungsbeispiel 5

In einem weiteren Ausführungsbeispiel der Erfindung konnten während einer (direkten) monochromen Mediatorsonden-(Mediator Probe) multiplex PCR jeweils Proben im roten, grünen und blauen Detektionskanal parallel analysiert werden. Hierfür wurden die Proben in einem entsprechenden digitalen PCR System, wie dem Stilla Naica System, amplifiziert und analysiert. Für jeden Detektionskanal wurden hierbei jeweils zwei, eine und keiner DNA-Zielsequenz zu einer Duplexreaktion (Reaktionsgemisch umfassend zwei verschiedene Reportermolekültypen, welche jeweils für eine der zwei DNA-Zielsequenzen spezifisch sind) zugegeben, sodass entweder beide Zielsequenzen gleichzeitig oder jeweils nur eine oder keine davon im jeweiligen Detektionskanal detektiert werden konnte.

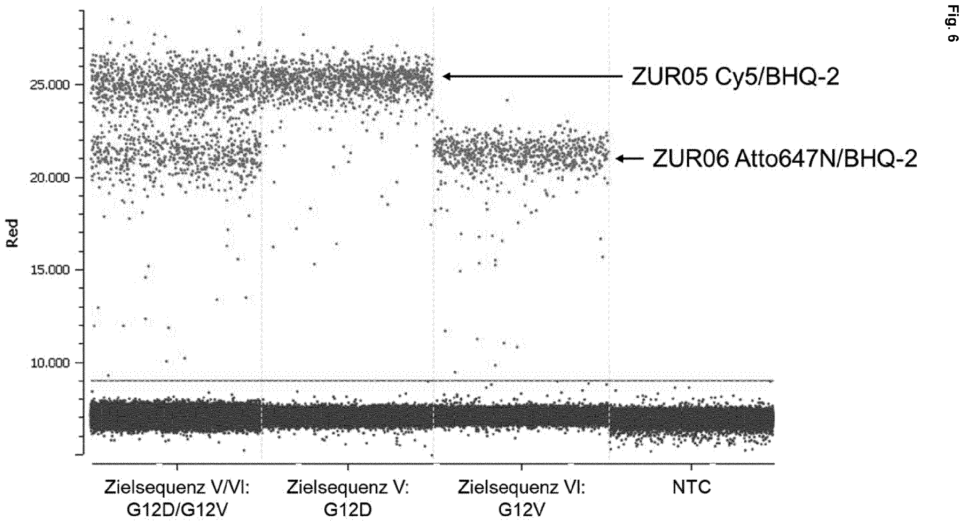

Figur 6 zeigt die Ergebnisse dieses Ausführungsbeispiels für die Verwendung von zwei verschiedenen Reporterkomplexen/Reportermolekülen im roten Detektionskanal in einer (direkten) monochromen Multiplexing-Analyse im Stilla Naica System. Dargestellt sind die Signale einer Duplexreaktion (Doppelreaktion), wobei im äußerst linken Datenraum die Signale, welche charakteristisch für die Zielsequenz 1 und Zielsequenz 2 (X-Achse) sind, dargestellt sind. Im mittleren Datenraum sind jeweils die Signale der Reaktionen bei einfacher Zugabe einer Zielsequenz dargestellt (Einzelreaktionen im Duplex-Reaktionsansatz), welche für die einzelne Zielsequenz 1 (X-Achse) bzw. Zielsequenz 2 (X-Achse) spezifisch sind, dargestellt. Im äußerst rechten Datenraum ist das gemessene Signal für die Negativkontrolle (NTC; X-Achse) dargestellt. Auf der Y-Achse ist die detektierte Signalintensität im roten Detektionskanal aufgeführt. Zur Signalgenerierung wurden zwei Typen von Zielsequenz-Unabhängigen-Reportermolekülen (ZUR05 und ZUR06) eingesetzt, entweder markiert mit Cy5/BHQ-2 oder BHQ-2/Atto-647N.

Die unterschiedlichen Signale der Reporterkomplexe/Reportermoleküle sind als distinkte Signalpopulationen im zweidimensionalen Datenraum gut voneinander differenzierbar, sodass jeweils eine spezifische Detektion beider Zielsequenzen möglich war.

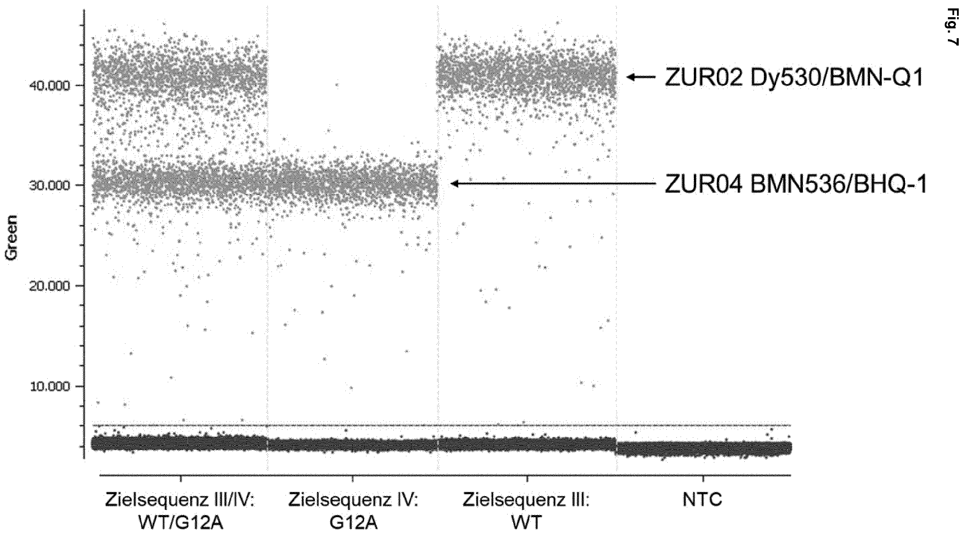

Figur 7 zeigt die Ergebnisse dieses Ausführungsbeispiels für die Verwendung von zwei verschiedenen Reporterkomplexen/Reportermolekülen im grünen Detektionskanal in einer (direkten) monochromen Multiplexing-Analyse im Stilla Naica System. Dargestellt sind die Signale einer Duplexreaktion (Doppelreaktion), wobei im äußerst linken Datenraum die detektierten Signale, welche spezifisch für die Zielsequenz 1 und Zielsequenz 2 (X-Achse) sind, dargestellt sind. Im mittleren Datenraum sind jeweils die beiden Einzelnachweise nach Zugabe von nur jeweils einer DNA-Zielsequenz für den einzelnen Nachweis der Zielsequenz 1 (X-Achse) bzw. Zielsequenz 2 (X-Achse) dargestellt. Im äußerst rechten Datenraum ist die Negativkontrolle (NTC; X-Achse) dargestellt. Auf der Y-Achse ist die Signalintensität im grünen Detektionskanal aufgeführt. Zur Signalgenerierung wurden zwei Typen von Zielsequenz-Unabhängigen-Reportermolekülen (ZUR02 und ZUR04) eingesetzt, entweder markiert mit Dy530 und BMN-Q1 oder mit BMN536 und BHQ-1.

Die unterschiedlichen Signale der Reporterkomplexe/Reportermoleküle sind als distinkte Signalpopulationen im zweidimensionalen Datenraum gut voneinander differenzierbar, sodass jeweils eine spezifische Detektion beider Zielsequenzen möglich war.

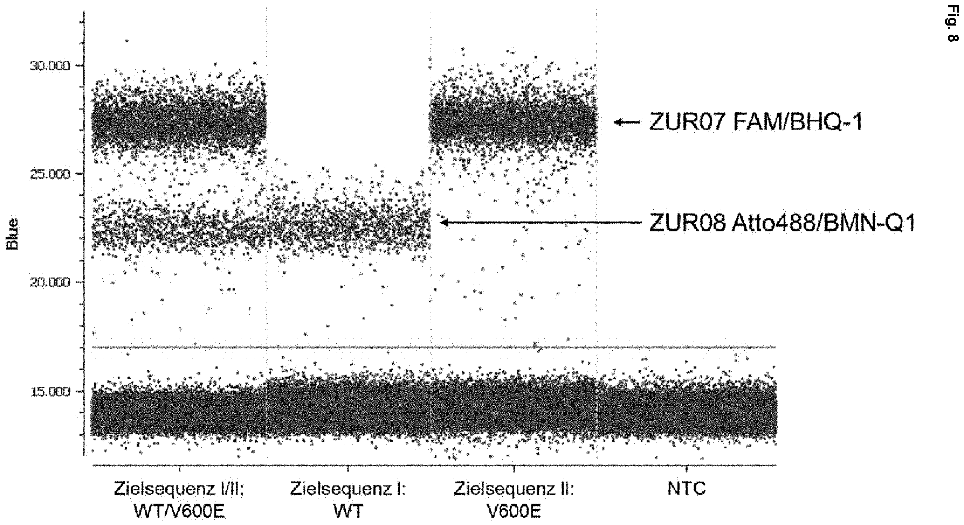

Figur 8 zeigt die Ergebnisse dieses Ausführungsbeispiels für die Verwendung von zwei verschiedenen Reporterkomplexen/Reportermolekülen im blauen Detektionskanal in einer (direkten) monochromen Multiplexing-Analyse im Stilla Naica System. Dargestellt sind die Signale einer Duplexreaktion (Doppelreaktion), wobei im äußerst linken Datenraum die Signale, welche für die Zielsequenz 1 und Zielsequenz 2 (X-Achse) spezifisch sind, dargestellt sind. Im mittleren Datenraum sind jeweils die beiden Einzelnachweise nach Zugabe von nur jeweils einer DNA-Zielsequenz, welche die Präsenz der einzelnen Zielsequenz 1 (X-Achse) bzw. Zielsequenz 2 (X-Achse) anzeigen, dargestellt. Im äußerst rechten Datenraum ist das Signal der Negativkontrolle (NTC; X-Achse) dargestellt. Auf der Y-Achse ist die Signalintensität im blauen Detektionskanal aufgeführt. Zur Signalgenerierung wurden zwei Typen von Zielsequenz-Unabhängigen-Reportermolekülen (ZUR07 und ZUR08) eingesetzt, entweder markiert mit FAM/BHQ-1 oder mit Atto488/BMN-Q1.

Die unterschiedlichen Signale der Reporterkomplexe/Reportermoleküle sind als distinkte Signalpopulationen im zweidimensionalen Datenraum gut voneinander differenzierbar, sodass jeweils eine spezifische Detektion beider Zielsequenzen möglich war.

### Ausführungsbeispiel 6

In einem weiteren Ausführungsbeispiel der Erfindung konnten während einer (direkten) monochromen Mediatorsonden-(Mediator Probe) multiplex PCR jeweils Proben im grünen und blauen Detektionskanal auf die Präsenz von 4 Zielsequenzen gleichzeitig analysiert werden. Hierfür wurden die Proben in einem entsprechenden digitalen PCR System, wie dem Stilla Naica System, amplifiziert und analysiert. In dieser "4-Plex Mediator Probe PCR" wurden vierZielsequenz-Unabhängige-Reportermoleküle, sowie vier Zielsequenz spezifische Mediatorsonden bereitgestellt, um die DNA-Zielsequenzen I - KRAS G12A (KRAS-Genmutation G12A), II - KRAS WT (KRAS-Wildtypgen), III - BRAF V600E (BRAF-Genmutation V600E) und IV- BRAF WT (BRAF-Wildtypgen) zu detektieren. Die Reportermoleküle für KRAS Zielgenvarianten (unterschiedliche BRAF oder KRAS Single Nucleotide Polymorphisms (SNP) oder Wild-Typ (WT) Varianten) erzeugten bei Aktivierung ein Signal im grünen Detektionskanal (BMN536 und Dy-530 Fluorophor), die Reportermoleküle für BRAF Zielgenvarianten erzeugten bei Aktivierung ein Signal im blauen Detektionskanal (FAM und Atto 488 Fluorophor).

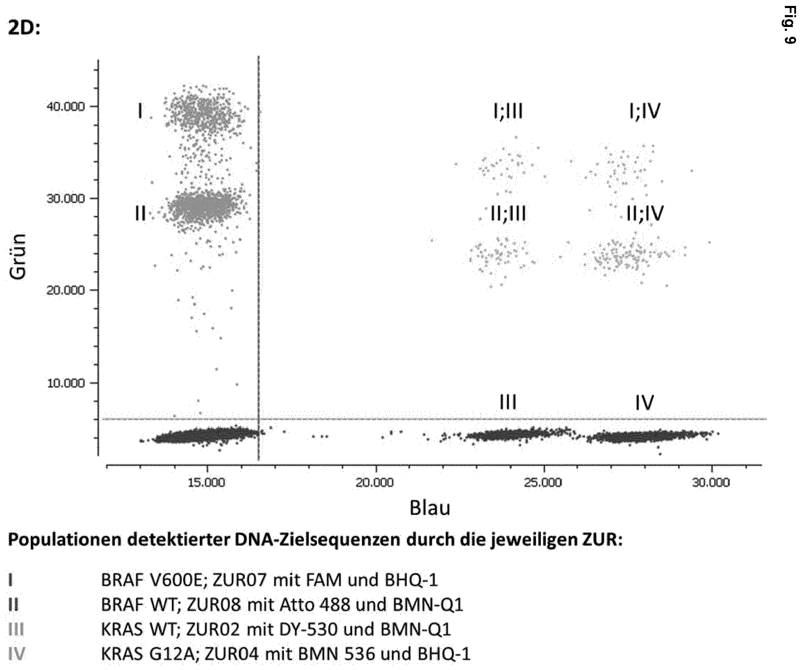

Figur 9 zeigt das Ergebnis dieser SNP-Analyse, wobei die einzeln in einer Partition detektierten Signale der Zielsequenzen I und II im linken oberen Quadrant des Datenraums dargestellt sind. Im rechten unteren Quadrant des Datenraums sind die einzeln in einer Partition detektierten Signale der Zielsequenzen III und IV dargestellt. Im rechten oberen Quadrant des Datenraums sind die gleichzeitig in einer Partition detektierten Signale von jeweils einer KRAS und einer BRAF Zielsequenz (also I+III, II+III, I+IV und II+IV) dargestellt. Auf derY-Achse ist die Signalintensität im grünen Detektionskanal (BMN536 und Dy-530 Fluorophor), auf der X-Achse die Signalintensität im blauen Detektionskanal (FAM und Atto 488 Fluorophor) aufgeführt.

Wie in Figur 9 zu erkennen ist, lassen sich mehrere Signalcluster je Detektionskanal detektieren, welche aktivierten, Zielsequenz-Unabhängigen-Reportermolekülen und somit den entsprechenden Zielsequenzen, zugeordnet werden können.

### Ausführungsbeispiel 7

In einem weiteren Ausführungsbeispiel der Erfindung konnten während einer (direkten) monochromen Mediatorsonden-(Mediator Probe) multiplex PCR jeweils Proben im grünen, roten und blauen Detektionskanal auf die Präsenz von 6 Zielsequenzen gleichzeitig analysiert werden. Hierfür wurden die Proben in einem entsprechenden digitalen PCR System, wie dem Stilla Naica System, amplifiziert und analysiert. In dieser "6-Plex Mediator Probe PCR" wurden sechs Zielsequenz-Unabhängige-Reportermoleküle, sowie sechs Zielsequenz-spezifische Mediatorsonden bereitgestellt, um die Zielsequenzen I - BRAF V600E (BRAF-Genmutation V600E), II - BRAF WT (BRAF-Wildtypgen), III - KRAS WT (KRAS-Wildtypgen), IV - KRAS G12A (KRAS-Genmutation G12A), V - KRAS G12D (KRAS-Genmutation G12D), VI- KRAS G12V (KRAS-Genmutation G12V) zu detektieren. Die Reportermoleküle für BRAF Zielgenvarianten BRAF V600E und WT erzeugten bei Aktivierung ein Signal im blauen Detektionskanal (FAM bzw. Atto 488 Fluorophor), die Reportermoleküle für KRAS WT und G12A Zielgenvarianten erzeugten bei Aktivierung ein Signal im grünen Detektionskanal (DY-530 bzw. BMN-536 Fluorophor) und die Reportermoleküle für KRAS G12D und G12V Zielgenvarianten erzeugten bei Aktivierung ein Signal im roten Detektionskanal (Cy5 bzw. Atto-647N Fluorophor).

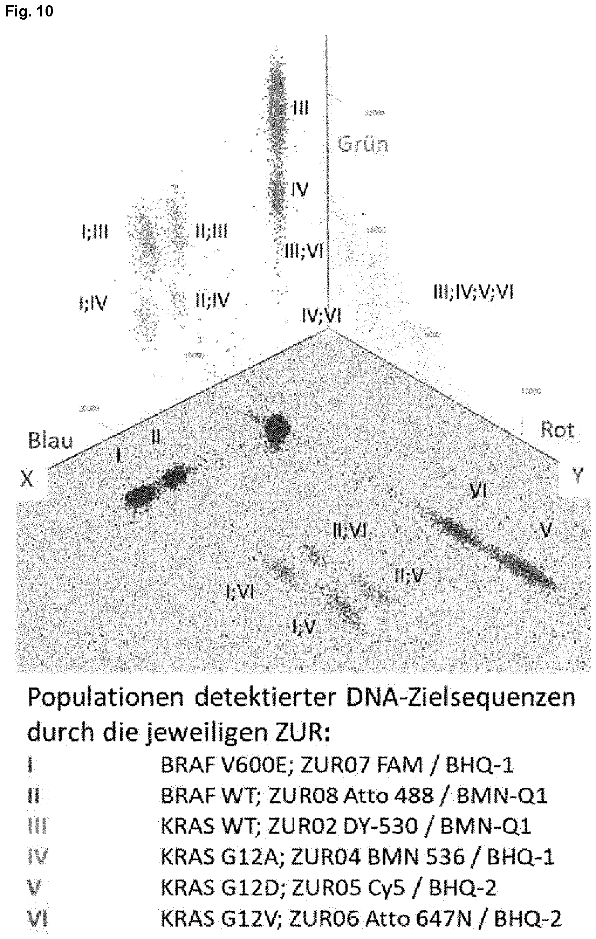

Figur 10 zeigt das Ergebnis dieser Analyse, wobei die einzeln in einer Partition detektierten Signale der Zielsequenzen I und II auf der X-Achse des Datenraums dargestellt sind. Auf der Y-Achse des Datenraums sind die einzeln in einer Partition detektierten Signale der Zielsequenzen V und VI dargestellt. Auf der Z-Achse des Datenraums sind die einzeln in einer Partition detektierten Signale der Zielsequenzen III und IV dargestellt. Im Datenraum sind zudem die gleichzeitig in einer Partition detektierten Signale von den jeweiligen KRAS bzw. BRAF Zielsequenzen dargestellt. Auf der Y-Achse ist die Signalintensität im roten Detektionskanal, auf der X-Achse die Signalintensität im blauen Detektionskanal und auf der Z-Achse die Signalintensität im grünen Detektionskanal aufgeführt.

Wie in Figur 10 zu erkennen ist, lassen sich mehrere Signalclusterje Detektionskanal detektieren, welche aktivierten, Zielsequenz-Unabhängigen-Reportermolekülen und somit den entsprechenden Zielsequenzen, zugeordnet werden können.

### Ausführungsbeispiel 8

Durch die Anwendung des beschriebenen Verfahrens ist es ebenfalls möglich, im höherdimensionalen Datenraum (>3 Dimensionen bei mehr als drei Detektionskanälen) eine Datenklassifizierung durchzuführen. Durch das beschriebene Verfahren kann die Anzahl an unterscheidbaren Zielsequenzen in einer digitalen PCR theoretisch mindestens gegenüber den zur Verfügung stehenden Detektionskanälen verdoppelt werden, sofern dies nicht per se durch geräteseitige technische Limitationen wie einem Crosstalk zwischen unterschiedlichen Detektionskanälen eingeschränkt ist:

| Anzahl geräteseitiger Detektionskanäle | Theoretische minimale Anzahl differenzierbarer Zielsequenzen |
|---|---|
| 1 | 2 |
| 2 | 4 |
| 3 | 6 |
| 4 | 8 |
| 5 | 10 |
| 6 | 12 |
| 7 | 14 |
| N | 2N |

Dem Fachmann sind hierbei entsprechende Verfahren bekannt, wie höherdimensionale Datenstrukturen klassifiziert werden können.

### FIGUREN

Die Erfindung wird weiter durch die folgenden Figuren beschrieben. Diese sollen den Umfang der Erfindung nicht einschränken, sondern stellen bevorzugte Ausführungsformen von Aspekten der Erfindung dar, die zur Veranschaulichung der hierin beschriebenen Erfindung bereitgestellt werden.
**Figur 1****:** Direktes monochromes Multiplexing in der digitalen PCR durch Einsatz von zwei Zielsequenz-Unabhänigen-Reportermolekülen ("ZUR") eines ersten (A) und eines zweiten (B) Typs mit jeweils unterschiedlicher Fluorophor-Markierung (dargestellt als Kreis oder Dreieck) mit einem Emissionsmaximum im roten Detektionskanal in einer Konfiguration, welche eine Signalidentifikation ermöglicht. Unterschiedliche Signalstärken der Fluoreszenz, welche charakteristisch für eine jeweilige Nukleinsäure-Zielsequenz sind, werden durch unterschiedlich große Kreise dargestellt. Durch den Einsatz Zielsequenz-Unabhängiger-Reportermoleküle kann im Gegensatz zu Zielsequenz spezifischen Taqman-Sonden effizienter ein Kontakt-Quenching (des mind. einen Fluorophors durch mind. einen Quencher) eingestellt werden, wodurch die Unterschiede in der Signalstärke unterschiedlicher Fluorophore im selben Detektionskanal verstärkt werden. Der mind. eine Quencher ist am 5'-Ende des Reportermoleküls lokalisiert ("5'-Quencher modification"). Jeder Typ der Reportermoleküle ("ZUR 1" unter (A) oder "ZUR 2" unter (B)) wird durch eine spezifische Mediator Sequenz ("Mediator 1" unter (A) oder "Mediator 2" unter (B)) gebunden und aktiviert, welche zuvor von der zugehörigen Mediator-Sonde ("Mediator Probe 1" unter (A) oder "Mediator Probe 2" unter (B)) abgespalten und/oder freigesetzt wurde.
**Figur 2****:** Der Graph stellt die Ergebnisse des Ausführungsbeispiels 3 durch einen eindimensionalen Plot (1-D-Plot) dar. Im Beispiel 2 wurden zwei Nukleinsäure-Zielsequenzen durch eine (direkte) monochrome ("Mediator Probe") Duplex-PCR (digitale PCR im Stilla Naica System) mittels zugehöriger spezifischer Zielsequenz-Unabhängiger-Reportermolekültypen eines ersten und eines zweiten Typs nachgewiesen. Die zwei unterschiedlichen Reportermolekül-Typen umfassten entweder den roten Fluorophor Cy5 oder den roten Fluorophor Atto 647N als Markierung, welche charakteristisch für die jeweilige Nukleinsäure-Zielsequenz waren und im roten Detektionskanal eines Stilla Naica Systems (digitales PCR-System) detektiert und analysiert wurden.
**Figur 3****:** Der Graph stellt die Ergebnisse des Ausführungsbeispiels 3 durch einen zweidimensionalen (2-D) Plot dar. Im Beispiel 2 wurden zwei Nukleinsäure-Zielsequenzen durch eine (direkte) monochrome ("Mediator Probe") Duplex-PCR (digitale PCR im Stilla Naica System) mittels zugehöriger spezifischer Zielsequenz-Unabhängiger-Reportermolekültypen eines ersten und eines zweiten Typs nachgewiesen. Die zwei unterschiedlichen Reportermolekül-Typen umfassten entweder den roten Fluorophor Cy5 oder den roten Fluorophor Atto 647N als Markierung, welche charakteristisch für die jeweilige Nukleinsäure-Zielsequenz waren und im roten Detektionskanal eines Stilla Naica Systems (digitales PCR-System) detektiert wurden. Dieser Plot stellt in einem 2-D Datenraum die spezifischen Signale der Reporterkomplexe/Reportermoleküle im roten Kanal ("Red", Y-Achse) dem Hintergrundsignal (Hintergrundrauschen) im bauen Detektionskanal ("Blue", X-Achse) i gegenüber. Einheit der Y-Achse: relative fluorescence units (RFU).
**Figur 4****:** Der Graph stellt die Ergebnisse des Ausführungsbeispiels 3 durch einen dreidimensionalen (3-D) Plot dar. Im Beispiel 2 wurden zwei Nukleinsäure-Zielsequenzen durch eine (direkte) monochrome ("Mediator Probe") Duplex-PCR (digitale PCR im Stilla Naica System) mittels zugehöriger spezifischer Zielsequenz-Unabhängiger-Reportermolekültypen eines ersten und eines zweiten Typs nachgewiesen. Die zwei unterschiedlichen Reportermolekül-Typen umfassten entweder den roten Fluorophor Cy5 oder den roten Fluorophor Atto 647N als Markierung, welche charakteristisch für die jeweilige Nukleinsäure-Zielsequenz waren und im roten Detektionskanal eines Stilla Naica Systems (digitales PCR-System) detektiert wurden. Dieser Plot stellt in einem 3-D Datenraum die spezifischen Signale der Reporterkomplexe/Reportermoleküle im roten Kanal ("Red", Y-Achse) dem Hintergrundsignal (Hintergrundrauschen) im blauen Detektionskanal ("Blue", X-Achse) und den Signalen einer Negativkontrolle (enthielt kein Zielsequenz) im grünen Detektionskanal ("Green", Z-Achse) gegenüber. Einheit der Achsen: relative fluorescence units (RFU).
**Figur 5****:** Dargestellt ist ein Schema des Versuchsdesigns und der verwendeten Reportermoleküle des Ausführungsbeispiels 4. Im Ausführungsbeispiel 4 erfolgt in einer digitalen PCR-Reaktion unter Verwendung von vier verschiedenen Typen von Zielsequenz-Unabhängigen-Reportermolekülen (ZUR1-ZUR4) mit unterschiedlichen Fluoreszenzmarkierungen ein direktes monochromes Multiplexing in zwei Detektionskanälen (z.B. roter Kanal (X-Achse) und grüner Kanal (Y-Achse)). Die vier verschiedenen Typen von Zielsequenz-Unabhängigen-Reportermolekülen (ZUR1-ZUR4) werden jeweils von einer spezifischen Mediatorsequenz (Mediator Typ 1-4) gebunden und aktiviert, wobei das generierte und detektierte Fluoreszenzsignal jeweils spezifisch für eine jeweilige Nukleinsäure-Zielsequenz ist.
**Figur 6****:** Dargestellt sind die Ergebnisse des Ausführungsbeispiels 5. Es wurden zwei verschiedenen Zielsequenz-Unabhängige-Reportermolekültypen im roten Detektionskanal in einer (direkten) monochromen Multiplexing-PCR Analyse im Stilla Naica System verwendet. Dargestellt sind die Signale einer Duplexreaktion (Doppelreaktion), wobei im äußerst linken Datenraum die Signale, welche die Präsenz der Zielsequenz 1 und Zielsequenz 2 (X-Achse) anzeigen, dargestellt sind. Im mittleren Datenraum sind jeweils die Signale der Einzelnachweise nach Zugabe von nur jeweils einer DNA-Zielsequenz), welche für die Zielsequenz 1 (X-Achse) und Zielsequenz 2 (X-Achse) spezifisch sind, dargestellt. Im äußerst rechten Datenraum ist das Signal der Negativkontrolle (NTC; X-Achse) dargestellt. Auf der Y-Achse ist die Signalintensität im roten Detektionskanal ("Red") aufgeführt. Zur Signalgenerierung wurden zwei Typen von Zielsequenz-Unabhängigen-Reportermolekülen (ZUR05 und ZUR06) eingesetzt, entweder markiert mit Cy5/BHQ-2 oder BHQ-2/Atto-647N. Einheit der Y-Achse: relative fluorescence units (RFU).
**Figur 7****:** Dargestellt sind die Ergebnisse dieses Ausführungsbeispiels 5 für die Verwendung von zwei verschiedenen Reporterkomplexen/Reportermolekülen im grünen Detektionskanal in einer (direkten) monochromen Multiplexing-Analyse im Stilla Naica System. Dargestellt sind die Signale einer Duplexreaktion (Doppelreaktion), wobei im äußerst linken Datenraum die Signale, welche für die Zielsequenz 1 und Zielsequenz 2 (X-Achse) spezifisch sind, dargestellt sind. Im mittleren Datenraum sind jeweils die beiden Einzelnachweise nach Zugabe von nur jeweils einer DNA-Zielsequenz für den Nachweis der einzelnen Zielsequenz 1 (X-Achse) bzw. Zielsequenz 2 (X-Achse) dargestellt. Im äußerst rechten Datenraum ist das Signal der Negativkontrolle (NTC; X-Achse) dargestellt. Auf der Y-Achse ist die Signalintensität im grünen Detektionskanal aufgeführt. Zur Signalgenerierung wurden zwei Typen von Zielsequenz-Unabhängigen-Reportermolekülen (ZUR02 und ZUR04) eingesetzt, entweder markiert mit Dy530/BMN-Q1 oder mit BMN536/BHQ-1. Einheit der Y-Achse: relative fluorescence units (RFU).
**Figur 8****:** Dargestellt sind die Ergebnisse dieses Ausführungsbeispiels 5 für die Verwendung von zwei verschiedenen Reporterkomplexen/Reportermolekülen im blauen Detektionskanal in einer (direkten) monochromen Multiplexing-Analyse im Stilla Naica System. Dargestellt sind die Signale einer Duplexreaktion (Doppelreaktion), wobei im äußerst linken Datenraum die Signale, welche für die Zielsequenz 1 und Zielsequenz 2 (X-Achse) spezifisch sind, dargestellt sind. Im mittleren Datenraum sind jeweils die Einzelnachweise nach Zugabe von nur jeweils einer DNA-Zielsequenz, welche die Präsenz der einzelnen Zielsequenz 1 (X-Achse) bzw. Zielsequenz 2 (X-Achse) anzeigen, dargestellt. Im äußerst rechten Datenraum ist das Signal der Negativkontrolle (NTC; X-Achse) dargestellt. Auf der Y-Achse ist die Signalintensität im blauen Detektionskanal aufgeführt. Zur Signalgenerierung wurden zwei Typen von Zielsequenz-Unabhängigen-Reportermolekülen (ZUR07 und ZUR08) eingesetzt, entweder markiert mit FAM/BHQ-1 oder mit Atto488/BMN-Q1. Einheit der Y-Achse: relative fluorescence units (RFU).
**Figur 9****:** Dargestellt ist das Ergebnis des Ausführungsbeispiels 6 wobei durch eine (direkte) monochrome Mediatorsonden-(Mediator Probe) multiplex PCR jeweils Proben im grünen und blauen Detektionskanal auf die Präsenz von 4 Zielsequenzen gleichzeitig analysiert wurden. Hierfür wurden die Proben in einem entsprechenden digitalen PCR-System (Stilla Naica System), amplifiziert und analysiert. In dieser "4-Plex Mediator Probe PCR" wurden spezifische Zielsequenz-Unabhängige-Reportermoleküle für die Zielsequenzen I - KRAS G12A (KRAS-Genmutation G12A), II - KRAS WT (KRAS-Wildtypgen), III - BRAF V600E (BRAF-Genmutation V600E) und IV- BRAF WT (BRAF-Wildtypgen) eingesetzt. Die Reportermoleküle für KRAS-Zielgenvarianten erzeugten bei Aktivierung ein Signal im grünen Detektionskanal (Y-Achse), die Reportermoleküle für BRAF Zielgenvarianten erzeugten bei Aktivierung ein Signal im blauen Detektionskanal (X-Achse). Im abgebildeten 2-D Plot (2-D Datenraum) sind die einzeln in einer Partition detektierten Signale der Zielsequenzen I und II im linken oberen Quadrant des Datenraums dargestellt sind. Im rechten unteren Quadrant des 2-D Datenraums sind die einzeln in einer Partition detektierten Signale der Zielsequenzen III und IV dargestellt. Im rechten oberen Quadrant des 2-D Datenraums sind die gleichzeitig in einer Partition detektierten Signale von jeweils einer KRAS und einer BRAF-Zielsequenz (also I+III, II+III, I+IV und II+IV) dargestellt. Auf der Y-Achse ist die Signalintensität im grünen Detektionskanal, auf der X-Achse die Signalintensität im blauen Detektionskanal aufgeführt. Einheit der Achsen: relative fluorescence units (RFU).
**Figur 10****:** Die Figur zeigt in einem dreidimensionalen (3-D) Plot die Ergebnisse des Ausführungsbeispiels 6 wobei mittels einer (direkten) monochromen Mediatorsonden-(Mediator Probe) multiplex PCR jeweils Proben im grünen, roten und blauen Detektionskanal auf die Präsenz von 6 Zielsequenzen gleichzeitig analysiert wurden. Hierfür wurden die Proben in einem entsprechenden digitalen PCR-System (Stilla Naica System) amplifiziert und analysiert. In dieser "6-Plex Mediator Probe PCR" wurden spezifische Zielsequenz-Unabhängige-Reportermoleküle (ZUR02, ZUR04-ZUR08) für die folgenden Zielsequenzen eingesetzt: I - BRAF V600E (BRAF-Genmutation V600E; ZUR07, Markierung: FAM/BHQ-1), II - BRAF WT (BRAF-Wildtypgen; ZUR08, Markierung: Atto 488/BMN-W1), III - KRAS WT (KRAS-Wildtypgen; ZUR02, Markierung: DY-530/BMN-Q1), IV - KRAS G12A (KRAS-Genmutation G12A; ZUR04, Markierung: Cy5/BHQ-1), V - KRAS G12D (KRAS-Genmutation G12D; ZUR05, Markierung: Cy5/BHQ-2), VI- KRAS G12V (KRAS-Genmutation G12V, ZUR06, Markierung: Atto 647N/BHQ-2). Die Reportermoleküle für BRAF Zielgenvarianten BRAF V600E und WT erzeugten bei Aktivierung ein Signal im blauen Detektionskanal (FAM bzw. Atto 488 Fluorophor), die Reportermoleküle für KRAS WT und G12A Zielgenvarianten erzeugten bei Aktivierung ein Signal im grünen Detektionskanal (DY-530 bzw. BMN-536 Fluorophor) und die Reportermoleküle für KRAS G12D und G12V Zielgenvarianten erzeugten bei Aktivierung ein Signal im roten Detektionskanal (Cy5 bzw. Atto-647N Fluorophor). Im 3-D Plot (3-D Datenraum) sind die einzeln in einer Partition detektierten Signale der Zielsequenzen I und II auf der X-Achse dargestellt. Auf der Y-Achse des 3-D Datenraums sind die einzeln in einer Partition detektierten Signale der Zielsequenzen V und VI dargestellt. Auf der Z-Achse des 3-D Datenraums sind die einzeln in einer Partition detektierten Signale der Zielsequenzen III und IV dargestellt. Im 3-D Datenraum sind zudem die gleichzeitig in einer Partition detektierten Signale von den jeweiligen KRAS bzw. BRAF-Zielsequenzen dargestellt. Auf der Y-Achse ist die Signalintensität im roten Detektionskanal, auf der X-Achse die Signalintensität im blauen Detektionskanal und auf der Z-Achse die die Signalintensität im grünen Detektionskanal aufgeführt. Einheit der Achsen: relative fluorescence units (RFU).

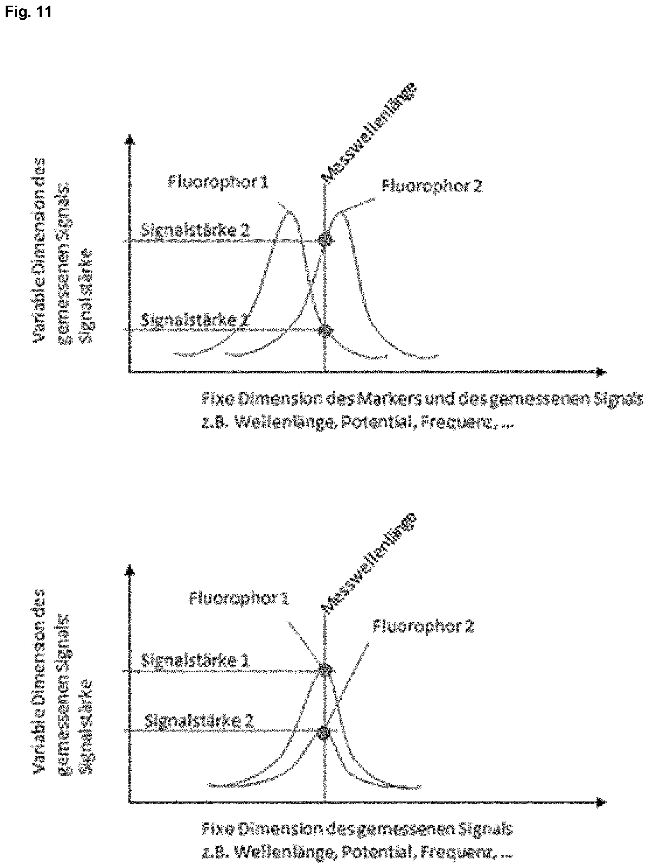

**Figur 11****:** Eine Möglichkeit unterschiedliche Signalstärken durch unterschiedliche Markierungen an Reportermolekülen zu erzeugen, ergibt sich in Ausführungsformen z.B. aus den physikalischen Moleküleigenschaften der Markierungen, welche durch ein Kontakt-Quenching nach der Aktivierung des Zielsequenz-unspezifischen Reportermoleküls verstärkt werden. In beiden Graphen sind auf der X-Achse die fixen Dimensionen einer Markierung ("Marker") und des gemessenen Signals dargestellt, z.B. Wellenlänge, Potential, Frequenz etc. Auf der Y-Achse sind die variablen Dimensionen des gemessenen Signals, hier die Signalstärke, dargestellt. Die Punkte zeigen im jeweiligen Graph den Schnittpunkt der jeweiligen Signalstärken 1 und 2 des Fluorophors 1 oder 2 und der jeweiligen Messwellenlänge an.

### LITERATURVERZEICHNIS

Faltin, Bernd; Wadle, Simon; Roth, Günter; Zengerle, Roland; Stetten, Felix von (2012): Media-tor probe PCR: a novel approach for detection of real-time PCR based on label-free primary probes and standardized secondary universal fluorogenic reporters. In: Clinical chemistry 58 (11), S. 1546-1556. DOI: 10.1373/clinchem.2012.186734.

Garcia-Murillas, Isaac; Schiavon, Gaia; Weigelt, Britta; Ng, Charlotte; Hrebien, Sarah; Cutts, Rosalind J. et al. (2015): Mutation tracking in circulating tumor DNA predicts relapse in early breast cancer. In: Science translational medicine 7 (302), 302ra133. DOI: 10.1126/scitranslmed.aab0021.

Hughesman, Curtis B.; Lu, X. J. David; Liu, Kelly Y. P.; Zhu, Yuqi; Poh, Catherine F.; Haynes, Charles (2016): A Robust Protocol for Using Multiplexed Droplet Digital PCR to Quantify Somatic Copy Number Alterations in Clinical Tissue Specimens. In: PloS one 11 (8), e0161274. DOI: 10.1371/journal.pone.0161274.

Kipf, Elena; Schlenker, Franziska; Borst, Nadine; Fillies, Marion; Kirschner-Schwabe, Renate; Zengerle, Roland et al. (2022): Advanced Minimal Residual Disease Monitoring for Acute Lym-phoblastic Leukemia with Multiplex Mediator Probe PCR. In: The Journal of molecular diagnos-tics : JMD 24 (1), S. 57-68. DOI: 10.1016/j.jmoldx.2021.10.001.

Lehnert, Michael; Kipf, Elena; Schlenker, Franziska; Borst, Nadine; Zengerle, Roland; Stetten, Felix von (2018): Fluorescence signal-to-noise optimisation for real-time PCR using universal reporter oligonucleotides. In: Anal. Methods 10 (28), S. 3444-3454. DOI: 10.1039/C8AY00812D.

Milbury, Coren A.; Zhong, Qun; Lin, Jesse; Williams, Miguel; Olson, Jeff; Link, Darren R.; Hutchison, Brian (2014): Determining lower limits of detection of digital PCR assays for cancerrelated gene mutations. In: Biomolecular detection and quantification 1 (1), S. 8-22. DOI: 10.1016/j.bdq.2014.08.001.

Pecoraro S., Berben G., Burns M., Corbisier P., De Giacomo M., De Loose M.,Dagand E., Dobnik D., Eriksson R.,Holst-Jensen A., Kagkli D. M., Kreysa J.,Lievens A., Mäde D., Mazzara M.,Paternö A., Peterseil V., Savini C.,Sovová T., Sowa S., Spilsberg B.: Overview and recom-mendations for the application of digital PCR. JRC Technical Report. Luxembourg: Publications Office of the European Union, 2019 (2019). Online verfügbar unter https://publications.jrc.ec.europa.eu/repository/handle/JRC115736.

Schlenker, Franziska; Kipf, Elena; Borst, Nadine; Hutzenlaub, Tobias; Zengerle, Roland; Stetten, Felix von; Juelg, Peter (2021a): Virtual Fluorescence Color Channels by Selective Photobleach-ing in Digital PCR Applied to the Quantification of KRAS Point Mutations. In: Analytical chemistry 93 (30), S. 10538-10545. DOI: 10.1021/acs.analchem.1c01488.

Schlenker, Franziska; Kipf, Elena; Deuter, Max; Höffkes, Inga; Lehnert, Michael; Zengerle, Ro-land et al. (2021b): Stringent Base Specific and Optimization-Free Multiplex Mediator Probe ddPCR for the Quantification of Point Mutations in Circulating Tumor DNA. In: Cancers 13 (22). DOI: 10.3390/cancers13225742.

Schuler, Friedrich; Trotter, Martin; Zengerle, Roland; Stetten, Felix von (2016): Monochrome Multiplexing in Polymerase Chain Reaction by Photobleaching of Fluorogenic Hydrolysis Probes. In: Analytical chemistry 88 (5), S. 2590-2595. DOI: 10.1021/acs.analchem.5b02960.

Whale, Alexandra S.; Huggett, Jim F.; Tzonev, Svilen (2016): Fundamentals of multiplexing with digital PCR. In: Biomolecular detection and quantification 10, S. 15-23. DOI: 10.1016/j.bdq.2016.05.002.

Corné, Julien; Le Du, Fanny; Quillien, Véronique; Godey, Florence; Robert, Lucie; Bourien, Héloïse et al. (2021): Development of multiplex digital PCR assays forthe detection of PIK3CA mutations in the plasma of metastatic breast cancer patients. In: Scientific reports 11 (1), S. 17316. DOI: 10.1038/s41598-021-96644-6.

Faltin, Bernd; Wadle, Simon; Roth, Günter; Zengerle, Roland; Stetten, Felix von (2012): Media-tor probe PCR: a novel approach for detection of real-time PCR based on label-free primary probes and standardized secondary universal fluorogenic reporters. In: Clinical chemistry 58 (11), S. 1546-1556. DOI: 10.1373/clinchem.2012.186734.

Garcia-Murillas, Isaac; Schiavon, Gaia; Weigelt, Britta; Ng, Charlotte; Hrebien, Sarah; Cutts, Rosalind J. et al. (2015): Mutation tracking in circulating tumor DNA predicts relapse in early breast cancer. In: Science translational medicine 7 (302), 302ra133. DOI: 10.1126/scitranslmed.aab0021.

Hughesman, Curtis B.; Lu, X. J. David; Liu, Kelly Y. P.; Zhu, Yuqi; Poh, Catherine F.; Haynes, Charles (2016): A Robust Protocol for Using Multiplexed Droplet Digital PCR to Quantify Somatic Copy Number Alterations in Clinical Tissue Specimens. In: PloS one 11 (8), e0161274. DOI: 10.1371/journal.pone.0161274.

Kipf, Elena; Schlenker, Franziska; Borst, Nadine; Fillies, Marion; Kirschner-Schwabe, Renate; Zengerle, Roland et al. (2022): Advanced Minimal Residual Disease Monitoring for Acute Lym-phoblastic Leukemia with Multiplex Mediator Probe PCR. In: The Journal of molecular diagnos-tics : JMD 24 (1), S. 57-68. DOI: 10.1016/j.jmoldx.2021.10.001.

Lehnert, Michael; Kipf, Elena; Schlenker, Franziska; Borst, Nadine; Zengerle, Roland; Stetten, Felix von (2018): Fluorescence signal-to-noise optimisation for real-time PCR using universal reporter oligonucleotides. In: Anal. Methods 10 (28), S. 3444-3454. DOI: 10.1039/C8AY00812D.

Madic, Jordan; Jovelet, Cécile; Lopez, Julien; André, Barbara; Fatien, Jean; Miran, Isabelle et al. (2018): EGFR C797S, EGFR T790M and EGFR sensitizing mutations in non-small cell lung cancer revealed by six-color crystal digital PCR. In: Oncotarget 9 (100), S. 37393-37406. DOI: 10.18632/oncotarget.26446.

Milbury, Coren A.; Zhong, Qun; Lin, Jesse; Williams, Miguel; Olson, Jeff; Link, Darren R.; Hutchison, Brian (2014): Determining lower limits of detection of digital PCR assays for cancerrelated gene mutations. In: Biomolecular detection and quantification 1 (1), S. 8-22. DOI: 10.1016/j.bdq.2014.08.001.

Pecoraro S., Berben G., Burns M., Corbisier P., De Giacomo M., De Loose M.,Dagand E., Dob-nik D., Eriksson R.,Holst-Jensen A., Kagkli D. M., Kreysa J.,Lievens A., Mäde D., Mazzara M.,Paternö A., Peterseil V., Savini C.,Sovová T., Sowa S., Spilsberg B.: Overview and recom-mendations for the application of digital PCR. JRC Technical Report. Luxembourg: Publications Office of the European Union, 2019 (2019). Online verfügbar unter https://publications.jrc.ec.europa.eu/repository/handle/JRC115736.

Schlenker, Franziska; Kipf, Elena; Borst, Nadine; Hutzenlaub, Tobias; Zengerle, Roland; Stetten, Felix von; Juelg, Peter (2021a): Virtual Fluorescence Color Channels by Selective Photobleach-ing in Digital PCR Applied to the Quantification of KRAS Point Mutations. In: Analytical chemistry 93 (30), S. 10538-10545. DOI: 10.1021/acs.analchem.1c01488.

Schlenker, Franziska; Kipf, Elena; Deuter, Max; Höffkes, Inga; Lehnert, Michael; Zengerle, Ro-land et al. (2021b): Stringent Base Specific and Optimization-Free Multiplex Mediator Probe ddPCR for the Quantification of Point Mutations in Circulating Tumor DNA. In: Cancers 13 (22). DOI: 10.3390/cancers13225742.

Schuler, Friedrich; Trotter, Martin; Zengerle, Roland; Stetten, Felix von (2016): Monochrome Multiplexing in Polymerase Chain Reaction by Photobleaching of Fluorogenic Hydrolysis Probes. In: Analytical chemistry 88 (5), S. 2590-2595. DOI: 10.1021/acs.analchem.5b02960.

Stilla Technologies: High multiplex, ultrasensitive EGFR detection using the EGFR 6-color Crys-tal Digital PCR™ kit. Stilla Technologies. Online verfügbar unter https://www.stillatechnolo-gies.com/wp-content/uploads/2021/12/StillaTechnologies-6-color-EGFR-kit-App-Note.pdf.

Whale, Alexandra S.; Huggett, Jim F.; Tzonev, Svilen (2016): Fundamentals of multiplexing with digital PCR. In: Biomolecular detection and quantification 10, S. 15-23. DOI: 10.1016/j.bdq.2016.05.002.

## Patentansprüche

1. Verfahren zum Nachweis von mindestens zwei Nukleinsäure-Zielsequenzen durch mindestens zwei Zielsequenz-Unabhängige-Reportermoleküle im selben Detektionskanal, umfassend die Schritte:
a. Bereitstellen von mindestens einer ersten und einer zweiten Nukleinsäure-Zielsequenz,
b. Bereitstellen von mindestens einer ersten und einer zweiten Mediatorsonde, jeweils umfassend ein Oligonukleotid,
wobei das Oligonukleotid der ersten Mediatorsonde eine Mediatorsequenz, und eine Sonden-Sequenz umfasst, wobei die Mediatorsequenz eine Affinität zu einem Zielsequenz-Unabhängigen-Reportermolekül eines ersten Typs besitzt und die Sonden-Sequenz eine Affinität zu einer ersten Nukleinsäure-Zielsequenz aufweist,
wobei das Oligonukleotid der zweiten Mediatorsonde eine Mediatorsequenz, und eine Sonden-Sequenz umfasst, wobei die Mediatorsequenz eine Affinität zu einem Zielsequenz-Unabhängigen-Reportermolekül eines zweiten Typs besitzt und die Sonden-Sequenz eine Affinität zu einer zweiten Nukleinsäure-Zielsequenz aufweist,
wobei die mindestens erste und zweite Mediatorsonden keine signalerzeugende Markierungen besitzen,
c. Bereitstellen von mindestens zwei Zielsequenz-Unabhängigen-Reportermolekülen mindestens eines ersten und eines zweiten Typs jeweils umfassend mindestens eine Markierung mit einem jeweils messbaren Signal im selben Detektionskanal, und eine Nukleinsäuresequenz, welche jeweils eine spezifische Affinität zu mindestens einer Mediatorsequenz besitzt,
wobei jeder Typ der mindesten zwei Zielsequenz-Unabhängigen-Reportermoleküle durch die jeweils mindestens eine Markierung dahingehend charakterisiert ist, dass diese jeweils eine Signalintensität besitzt, welche von den Signalintensitäten der Markierungen aller anderen Zielsequenz-Unabhängigen-Reportermolekül Typen unterscheidbar ist und eine direkte Zuordnung zu der jeweiligen Nukleinsäure-Zielsequenz ermöglicht,
d. Durchführen einer Nukleinsäure-Nachweisreaktion, wobei mindestens eine Mediatorsequenz mindestens einer ersten Mediatorsonde freigesetzt wird, wenn deren Sonden-Sequenz an mindestens eine erste Nukleinsäure-Zielsequenz bindet,
wobei die mindestens eine freigesetzte Mediatorsequenz an mindestens ein Zielsequenz-Unabhängige-Reportermolekül eines ersten Typs bindet, wobei durch die mindestens eine Markierung ein Signal erzeugt wird, welches durch seine Signalintensität und/oder Emissionsspektrum charakteristisch für die erste dem jeweiligen Zielsequenz-Unabhängigen Reporter zugeordneten Nukleinsäure-Zielsequenz ist, an welche die mindestens eine Sonden-Sequenz der mindestens einen ersten Mediator-sonde gebunden hat,
e. Detektion des unter Schritt d. erzeugten Signals, umfassend die Detektion des Signals in einem Detektionskanal und/oder eine Analyse des Signals, der Signalstärke und/oder des Emissionsspektrums des Signals.

2. Verfahren gemäß Anspruch 1, wobei es sich bei der mindestens einen Markierung eines Zielsequenz-Unabhängigen-Reportermoleküls um mindestens ein Fluorophor und/oder mindestens einen Quencher handelt.

3. Verfahren gemäß einem der vorherigen Ansprüche, wobei die Analyse des Signals, der Signalstärke und/oder des Emissionsspektrums des Signals unter Punkt e. die Darstellung und/oder Analyse des detektierten Signals abhängig von der Signalstärke und/oder dem Detektionskanal und/oder des Emissionsspektrums in einem durch die ausgewerteten Detektionskanäle aufgespannten Datenraum umfasst.

4. Verfahren gemäß einem der vorherigen Ansprüche, wobei unterschiedliche Zielsequenz-Unabhängige-Reportermolekül Typen sich durch die Signalintensität und/oder das Emissionsspektrum ihrer mindestens einen Markierung unterscheiden.

5. Verfahren gemäß einem der vorherigen Ansprüche, wobei die mindestens eine Markierung eines Zielsequenz-Unabhängigen-Reportermoleküls mindestens zwei Fluorophore und/oder zwei Quencher mit gleicher oder unterschiedlichem Emissionsspektrum und/oder gleicher oder unterschiedlicher Signalintensität umfasst.

6. Verfahren gemäß einem der vorherigen Ansprüche, wobei die mindestens eine Markierung eines Zielsequenz-Unabhängigen-Reportermoleküls mindestens ein Fluorophor und mindestens einen Quencher umfasst,
wobei zwischen dem mindestens einen Fluorophor und dem mindestens einen Quencher bevorzugt ein Kontakt-Quenching stattfindet, solange keine Mediatorsequenz am entsprechenden Zielsequenz-Unabhängigen-Reportermolekül bindet, oder solange eine gebundene Mediatorsequenz während der Nukleinsäure-Nachweisreaktion nicht verlängert wurde.

7. Verfahren gemäß einem der vorherigen Ansprüche, wobei die Markierung des mindestens einen Zielsequenz-Unabhängigen-Reportermoleküls mindestens zwei komplementäre gegenüberliegende Nukleobasen mit jeweils mindestens einer Markierung oder mindestens zwei um eine Basenposition gegenüber einer komplementären Basenpaarung versetzte gegenüberliegende Basen mit jeweils mindestens eine Markierung umfasst.

8. Verfahren gemäß einem der vorherigen Ansprüche, wobei in Schritt d.-e. n unterschiedliche Nukleinsäure-Zielsequenzen durch n unterschiedliche Zielsequenz-Unabhängige-Reportermolekül Typen indirekt detektiert werden, wobei die Detektion des in Schritt d. erzeugten Signals in k Detektionskanälen stattfindet,
wobei n > k ist und n ≥ 2 ist, und
wobei mindestens zwei unterschiedliche Zielsequenz-Unabhängige-Reportermolekül Typen in Schritt e. im gleichen Detektionskanal detektiert und/oder bei der Darstellung unter Punkt e. im gleichen Bereich eines Datenraums dargestellt werden.

9. Verfahren gemäß einem der vorherigen Ansprüche, wobei das Signal der Markierung eines Zielsequenz-Unabhängigen-Reportermoleküls durch die Spaltung und/oder Auftrennung des Zielsequenz-Unabhängigen-Reportermolekül und/oder durch räumliche Trennung des mindestens einen Fluorophor und des mindestens einen Quencher erzeugt wird.

10. Verfahren gemäß einem der vorherigen Ansprüche, wobei ein Zielsequenz-Unabhängiges-Reportermolekül ein Oligonukleotid oder ein Oligonukleotid-Komplex ist.

11. Verfahren gemäß einem der vorherigen Ansprüche, wobei die Nukleinsäure-Nachweisreaktion unter Schritt d. ein Amplifikationsverfahren für DNA und/oder cDNA umfasst.

12. Verfahren gemäß des vorherigen Anspruches, wobei die Nukleinsäure-Nachweisreaktion unter Schritt d. eine PCR, RT-PCR, RPA oder LAMP ist, und wobei im Rahmen der DNA-Amplifikation durch eine enzymatische Aktivität eines Biomoleküls eine Mediatorsequenz einer an eine Ziel-Nukleinsäure gebundene Mediatorsonde freigesetzt wird, welche darauf an ein Zielsequenz-Unabhängiges-Reportermolekül bindet, sodass ein Signal generiert wird.

13. Verfahren gemäß einem der beiden vorherigen Ansprüche, wobei die Detektion unter Schritt e. im Rahmen einer digitalen Amplifikation und/oder Signalgenerierung erfolgt.

14. Verfahren gemäß einem der vorherigen Ansprüche, wobei es sich bei den Zielsequenz-Unabhängigen-Reportermolekülen um Universal Reporter und/oder modulare Reporterkomplexe und bei der mindestens einen freigesetzten Mediatorsequenz um eine Komponente einer Mediator Probe PCR oder Mediator Displacement LAMP handelt.

15. Verfahren gemäß einem der vorherigen Ansprüche, wobei mindestens eine Nukleinsäure-Zielsequenz aus einer Konvertierung eines anderen Biomoleküls in DNA-Sequenzinformationen entstanden ist.

16. Kit, für die Durchführung des Verfahrens gemäß einem der vorherigen Ansprüche, umfassend:
- mindestens ein Zielsequenz-Unabhängiges-Reportermolekül eines ersten Typs umfassend mindestens eine Markierung,
- mindestens ein Zielsequenz-Unabhängiges-Reportermolekül eines zweiten Typs umfassend mindestens eine Markierung,
- mindestens eine erste Mediatorsonde, deren Mediatorsequenz eine Affinität für das mindestens eine Zielsequenz-Unabhängige-Reportermolekül eines ersten Typs besitzt, und deren Oligonukleotidsequenz eine Affinität zu einer ersten Nukleinsäure-Zielsequenz aufweist,
- mindestens eine zweite Mediatorsonde, deren Mediatorsequenz eine Affinität für das mindestens eine Zielsequenz-Unabhängige-Reportermolekül eines zweiten Typs besitzt, und deren Oligonukleotidsequenz eine Affinität zu einer zweiten Nukleinsäure-Zielsequenz aufweist,
- optional mindestens ein Puffer,
- optional eine Polymerase
- optional eine Reverse-Transkriptase
- optional mindestens ein PCR-Primer.
